(19) Europäisches Patentamt
European Patent Office
Office européen des brevets

(11) Veröffentlichungsnummer: **0 072 961**
**B1**

(12) # EUROPÄISCHE PATENTSCHRIFT

(45) Veröffentlichungstag der Patentschrift: **10.07.85**

(51) Int. Cl.⁴: **C 07 D 401/12**, C 07 D 409/12, C 07 D 231/56, A 61 K 31/415, A 61 K 31/495

(21) Anmeldenummer: **82107194.1**

(22) Anmeldetag: **09.08.82**

(54) **1-Phenylindazol-3-on-Verbindungen sowie Verfahren und Zwischenprodukte zu ihrer Herstellung und diese Verbindungen enthaltende Arzneimittel.**

(30) Priorität: **20.08.81 DE 3132916**

(43) Veröffentlichungstag der Anmeldung: **02.03.83 Patentblatt 83/9**

(45) Bekanntmachung des Hinweises auf die Patenterteilung: **10.07.85 Patentblatt 85/28**

(84) Benannte Vertragsstaaten: **AT BE CH DE FR GB IT LI LU NL SE**

(56) Entgegenhaltungen: **Chemical Abstracts Band 89, Nr. 21 20. November 1978 Columbus, Ohio, USA L. BAIOCCHI et al. "Synthesis, properties, and reactions of 1H-Indazol-3-ols and 1,2-dihydro-3H-indazol-3-ones" Seite 597, Spalte 1, Abstract Nr. 179882b**

(73) Patentinhaber: **Kali-Chemie Pharma GmbH, Hans-Böckler-Allee 20 Postfach 220, D-3000 Hannover 1 (DE)**

(72) Erfinder: **Heinemann, Henning Dipl.-Chem., Dr.rer.nat., Berglusstrasse 18, D-3000 Hannover 51 (DE)**
Erfinder: **Jasserand, Daniel, 2, Allée d'Andrezieux, F-75018 Paris (FR)**
Erfinder: **Milkowski, Wolfgang Dipl.-Chem., Dr.rer.nat., Fasanenweg 13, D-3167 Burgdorf (DE)**
Erfinder: **Yavordios, Dimitri, 536, Av. Dubanchet, F-01400 Châtillon s/Chalaronne (FR)**
Erfinder: **Zeugner, Horst Dipl.-Chem., Dr.rer.nat., Havelweg 10, D-3000 Hannover 73 (DE)**

(74) Vertreter: **Lauer, Dieter, Dr., c/o Kali-Chemie AG Postfach 220 Hans-Böckler-Allee 20, D-3000 Hannover 1 (DE)**

EP 0 072 961 B1

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

BUNDESDRUCKEREI BERLIN

## Beschreibung

Die vorliegende Erfindung betrifft neue 2-Piperazinoalkyl-1-phenylindazol-3-on-Verbindungen und deren Salze sowie diese Verbindungen enthaltende pharmazeutische Zubereitungen und Verfahren und Zwischenprodukte zur Herstellung dieser Verbindungen. Aus einer Arbeit über 1 H-Indazol-3-ole und 1,2-Dihydro-3 H-indazol-3-one von L. Baiocchi et al. (Synthesis, 1978, 633—648) ist die Herstellung von 1-Phenyl-2-(3-chlorpropyl)-1,2-dihydro-3 H-indazol-3-on bekannt.

Der Erfindung liegt die Aufgabe zugrunde, neue in 2-Stellung substituierte 1-Phenylindazol-3-on-Verbindungen mit wertvollen pharmakologischen Eigenschaften sowie Verfahren zu ihrer Herstellung bereitzustellen.

Es wurde nun gefunden, daß die erfindungsgemäßen neuen 1-Phenylindazol-3-on-Verbindungen wertvolle pharmakologische Eigenschaften, insbesondere ausgeprägte antiallergische Eigenschaften, sowie daneben auch cardiovasculäre und psychopharmakologische Eigenschaften, besitzen und ein vorteilhaftes Wirkungsprofil mit guter therapeutischer Breite und geringer Toxizität aufweisen. Aufgrund dieser Eigenschaften sind die neuen Verbindungen als Arzneimittel zur Behandlung von allergischen Erkrankungen wie beispielsweise allergisch bedingtem Asthma oder Heuschnupfen geeignet.

Die vorliegende Erfindung betrifft daher neue 1-Phenylindazol-3-on-Verbindungen der allgemeinen Formel I

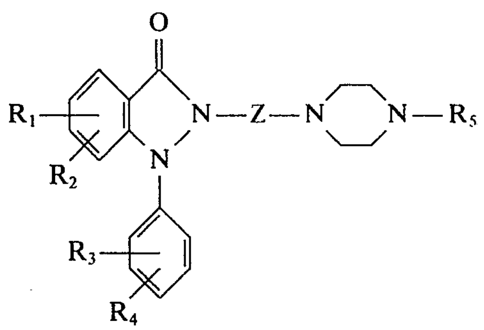

(I)

worin

$R_1$ Wasserstoff, $C_1$—$C_4$-Alkyl, $C_1$—$C_4$-Alkoxy, Halogen oder Trifluormethyl bedeutet,
$R_2$ Wasserstoff, $C_1$—$C_4$-Alkyl, $C_1$—$C_4$-Alkoxy oder Halogen bedeutet,
$R_3$ Wasserstoff, $C_1$—$C_4$-Alkyl, $C_1$—$C_4$-Alkoxy, Halogen oder Trifluormethyl bedeutet,
$R_4$ Wasserstoff, $C_1$—$C_4$-Alkyl, $C_1$—$C_4$-Alkoxy oder Halogen bedeutet,
Z eine Alkylengruppe mit 2—6 Kohlenstoffatomen bedeutet,
$R_5$ Pyridyl, welches unsubstituiert oder durch $C_1$—$C_4$-Alkyl, Halogen oder $C_1$—$C_4$-Alkoxy monosubstituiert ist, oder Thienyl oder eine gegebenenfalls substituierte Phenylgruppe a bedeutet

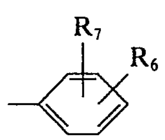

(a)

worin

$R_6$ Wasserstoff, $C_1$—$C_4$-Alkyl, $C_1$—$C_4$-Alkoxy, Hydroxy, Halogen, Trifluormethyl oder $C_1$—$C_4$-Alkanoyloxy bedeutet,
$R_7$ Wasserstoff, $C_1$—$C_4$-Alkyl, $C_1$—$C_4$-Alkoxy oder Halogen bedeutet oder
$R_6$ und $R_7$ an benachbarte Kohlenstoffatome gebunden sind und zusammen Methylendioxy oder Äthylendioxy bedeuten,

sowie deren Säureadditionssalze.

Sofern in den Verbindungen der Formel I die Substituenten $R_1$ bis $R_4$ und die in dem Rest $R_5$ enthaltenen Substituenten eine niedere Alkylgruppe enthalten, kann diese gerade oder verzweigt sein und 1 bis 4 Kohlenstoffatome enthalten. So kommen insbesondere Methyl, Äthyl, n-Propyl, Isopropyl, n-Butyl oder tert.-Butyl, bevorzugt Methyl, Äthyl, n-Propyl und Isopropyl in Frage. Insbesondere bei Disubstitution an den Phenylringen sind als Alkyl Methyl oder Äthyl, insbesondere Methyl, bevorzugt. Niedermolekulare Alkoxysubstituenten stellen vorzugsweise Methoxy oder Äthoxy dar.

Als Halogensubstituenten kommen insbesondere Fluor, Chlor oder Brom in Frage. Falls $R_1$ Trifluormethyl bedeutet, stellt $R_2$ vorzugsweise Wasserstoff dar. Im Falle von Halogen und/oder Alkyl bzw. und/oder Alkoxysubstituenten ist Mono- oder Disubstitution zweckmäßig. Bevorzugte Positionen für die Substituenten $R_1$ und $R_2$ sind die 5- und 6-Stellung. Falls eine Phenylgruppe a durch Trifluormethyl

substituiert ist, ist Monosubstitution bevorzugt. Im Falle von Halogen und/oder Alkyl bzw. und/oder Alkoxysubstituenten ist Mono- oder Disubstitution zweckmäßig.

Die Gruppe Z stellt eine gerade oder verzweigte Alkylenkette mit 2 bis 6 Kohlenstoffatomen dar, wobei Alkylenketten mit 2 bis 4 Kohlenstoffatomen bevorzugt sind.

Falls $R_5$ für eine Pyridyl-Gruppe steht, kann diese in 2-, 3- oder 4-Stellung, insbesondere in 2-Stellung, mit dem Restmolekül verbunden sein. Die Pyridyl-Gruppe kann unsubstituiert oder durch einen der vorgehend genannten Substituenten, insbesondere niederes Alkyl oder Alkoxy, vorzugsweise Methyl oder Methoxy substituiert sein.

Falls $R_5$ für eine Thienyl-Gruppe steht, kann diese 2- oder 3-Stellung, vorzugsweise in 2-Stellung, mit dem Restmolekül verbunden sein.

Erfindungsgemäß werden die neuen 1-Phenylindazol-3-on-Verbindungen der Formel I und deren Säureadditionssalze erhalten, indem man in an sich bekannter Weise

a) Verbindungen der Formel II a

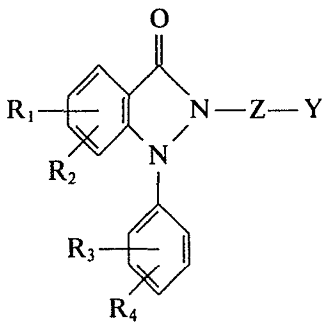

(IIa)

worin $R_1$, $R_2$, $R_3$, $R_4$ und Z obige Bedeutung besitzen, und Y einen aminolytisch abspaltbaren Rest bedeutet, mit einer Verbindung der Formel V

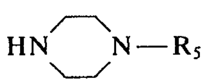

(V)

worin $R_5$ obige Bedeutung besitzt, umsetzt, oder

b) zur Herstellung von Verbindungen der Formel I a

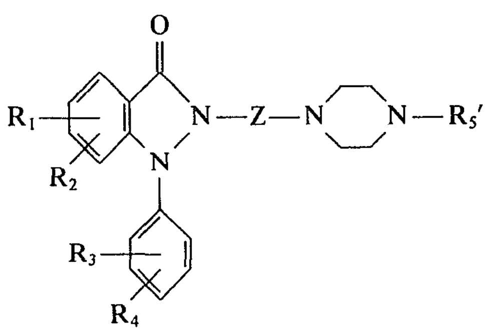

(Ia)

worin Z, $R_1$, $R_2$, $R_3$ und $R_4$ obige Bedeutung besitzen und $R_5'$ für eine substituierte Phenylgruppe a' steht

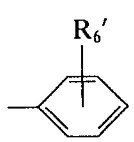

(a')

worin $R_6'$ ortho- oder paraständig ist und $CF_3$ bedeutet, Verbindungen der Formel III

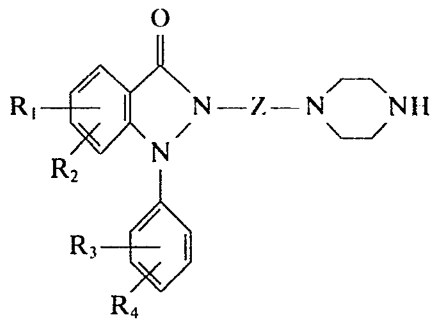

(III)

worin $R_1$, $R_2$, $R_3$, $R_4$ und Z obige Bedeutung besitzen, mit Verbindungen der Formel IV

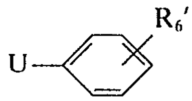

(IV)

worin $R_6'$ obige Bedeutung besitzt und U Halogen bedeutet, umsetzt und gegebenenfalls freie Verbindungen der Formel I in ihre Säureadditionssalze überführt oder die Säureadditionssalze in die freien Verbindungen der Formel I überführt.

Die Umsetzung der Verbindungen der Formel II a mit Verbindungen der Formel V gemäß Verfahrensvariante a) kann nach an sich zur Alkylierung von Aminen üblichen Methoden ausgeführt werden.

Die Umsetzung wird zweckmäßigerweise bei erhöhter Temperatur, beispielsweise bei Temperaturen zwischen 50 und 150°C, insbesondere 90 und 150°C, unter basischen Bedingungen durchgeführt. Als aminolytisch abspaltbare Reste in Verbindungen der Formel II a kommen insbesondere Halogene wie Chor, Brom oder Jod, vorzugsweise Chlor oder Brom, in Frage sowie auch organische Sulfonsäurereste, insbesondere Reste von Niederalkansulfonsäuren wie z. B. Methansulfonsäure oder Äthansulfonsäure oder von aromatischen Sulfonsäuren, insbesondere Benzolsulfonsäuren oder durch niederes Alkyl substituierte Benzolsulfonsäuren, z. B. Toluolsulfonsäuren, oder durch Halogen substituierte Benzolsulfonsäuren, wie z. B. Bromsulfonsäuren. Zweckmäßigerweise wird die Reaktion in einem unter den Reaktionsbedingungen inerten organischen Lösungsmittel durchgeführt. Als Beispiele geeigneter Lösungsmittel seien genannt aromatische Kohlenwasserstoffe wie Benzol, Toluol oder Xylol, cyclische Äther wie Dioxan, Dimethylformamid, 1,3-Dimethyl-2-imidazolidinon, Hexamethylphosphortriamid, Sulfolan, Dimethylsulfoxid, Tetramethylharnstoff oder niedere Alkanole wie beispielsweise Isopentanol. Gewünschtenfalls kann die Umsetzung der Verbindungen der Formel II a mit Verbindungen der Formel V jedoch auch ohne Lösungsmittel in der Schmelze stattfinden. Zweckmäßig kann man die Reaktion unter Zusatz einer organischen oder anorganischen Base durchführen. Man kann jedoch auch einen Überschuß der Verbindung der Formel V verwenden und diesen als interne Base benutzen. Geeignete anorganische Basen sind insbesondere Alkalimetallcarbonte oder -bicarbonate wie Natriumcarbonat, Natriumbicarbonat oder Kaliumcarbonat. Als organische Basen sind tertiäre organische Amine geeignet, insbesondere tertiäre Niederalkylamine wie Triäthylamin n-Tripropylamin, n-Tributylamin oder 1,4-Dimethylpiperazin.

Falls die Verbindungen der Formel II a oder V als Substituenten freie Hydroxy-Gruppen enthalten, werden diese zweckmäßigerweise während der Umsetzung in an sich bekannter Weise mit einer Schutzgruppe versehen. Geeignete nach der Reaktion leicht wieder abspaltbare Schutzgruppen sind z. B. bekannt aus E. McOmie ›Protective Groups in Organic Chemistry‹ Plenum Press (1971). Beispielsweise eignen sich zum Schutze einer Hydroxylfunktion Äther, insbesondere Tetrahydropyranyläther. Diese Schutzgruppen können nach der Umsetzung in bekannter Weise leicht wieder entfernt werden.

Die Umsetzung von Verbindungen der Formel III mit Verbindungen der Formel IV kann ebenfalls auf an sich bekannte Weise unter zur Alkylierung von Aminen üblichen Bedingungen, beispielsweise den vorstehend für die Umsetzung von Verbindungen der Formel II a mit Verbindungen der Formel V genannten Bedingungen, erfolgen. Die substituierten halogenierten Phenylverbindungen sind durch die Anwesenheit eines Substituenten zweiter Ordnung genügend aktiviert, um zur Umsetzung mit dem Piperazinderivat der Formel III befähigt zu sein.

Die Verbindungen der Formel I können auf an sich bekannte Weise aus dem Reaktionsgemisch isoliert und gereinigt werden. Säureadditionssalze können in üblicher Weise in die freien Basen überführt werden und diese gewünschtenfalls in bekannter Weise in pharmakologisch verträgliche Säureadditionssalze überführt werden.

Als pharmakologisch annehmbare Säureadditionssalze der Verbindungen der Formel I eignen sich beispielsweise deren Salze mit Chlorwasserstoffsäure, Bromwasserstoffsäure, Schwefelsäure, Phosphorsäure, Methansulfonsäure, Äthansulfonsäure, Benzolsulfonsäure, p-Toluolsulfonsäure, Zitronensäure, Essigsäure, Milchsäure, Bernsteinsäure, Maleinsäure, Fumarsäure, Apfelsäure, Weinsäure, Benzoesäure, Phenylessigsäure oder Mandelsäure.

Die Verbindungen der Formel I enthalten 2 oder, falls $R_5$ eine gegebenenfalls substituierte Pyridylverbindung bedeutet, auch 3 basische Zentren und können somit Säureadditionssalze mit 1, 2 oder 3 Äquivalenten Säure bilden. Zur Herstellung von pharmazeutischen Zubereitungen eignen sich insbesondere monosaure Salze. Salze, welche mehrere Äquivalente Säure enthalten, können gewünschtenfalls in an sich bekannter Weise in monosaure Salze überführt werden, z. B. durch Überführen in die freie Base und anschließende Umsetzung der Base mit einer äquivalenten Menge Säure.

Erfindungsgemäße Verbindungen der Formel I, worin Z eine verzweigte Alkylengruppe darstellt, werden bei der Synthese in Form ihrer Racemate erhalten. Unter den Schutz der vorliegenden Erfindung fallen sowohl die racemischen Gemische wie auch die optisch aktiven Formen dieser Verbindungen. Die optisch aktiven Verbindungen können aus den racemischen Gemischen in an sich bekannter Weise durch Umsetzung mit geeigneten optisch aktiven Säuren wie beispielsweise Weinsäure, O,O'-Dibenzoylweinsäure, Mandelsäure, Di-O-Isopropyliden-2-oxo-L-gulonsäure, und anschließende fraktionierte Kristallisation der gewonnenen Salze in ihre optisch aktiven Antipoden aufgetrennt werden (siehe z. B. Tetrahedron 33 (1977) Seiten 2725 bis 2736).

Verbindungen der Formel II mit Ausnahme solcher Verbindungen, worin $R_1$—$R_4$ Wasserstoff bedeuten und Z—Y''-Gruppe Cl-Propyl oder Cl-Äthyl bedeutet, sind in der Literatur bisher noch nicht beschrieben worden und stellen neue, wertvolle Zwischenprodukte für die Herstellung von pharmakologisch aktiven Verbindungen, beispielsweise den Verbindungen der Formel I, dar.

Verbindungen der Formel II können nach an sich bekannten Verfahren erhalten werden, indem man Alkalimetallsalze von 1-Phenyl-1,2-dihydro-3H-indazol-3-on-Verbindungen der allgemeinen Formel VI

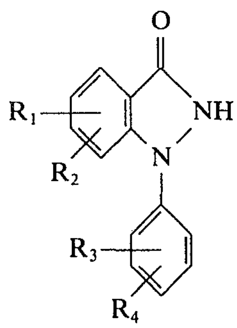

(VI)

worin $R_1$, $R_2$, $R_3$ und $R_4$ obige Bedeutung besitzen, mit Verbindungen der Formel VII

$$Y—Z—Y' \qquad (VII)$$

worin Z und Y obige Bedeutung besitzen und Y' eine geschützte Hydroxygruppe oder einen aminolytisch abspaltbaren Rest Y bedeutet, umsetzt. Als aminolytisch abspaltbare Reste Y kommen insbesondere Chlor, Brom, Jod oder reaktive Säurereste, beispielsweise die vorgehend genannten organischen Sulfonsäurereste in Frage. Y' stellt vorzugsweise Chlor, Brom oder eine geschützte Hydroxygruppe dar. Zum Schutze der Hydroxyfunktion während der Umsetzung können gebräuchliche Schutzgruppen wie beispielsweise Äther, insbesondere der Tetrahydropyranyläther, verwendet werden. Geeignete Schutzgruppen für die Hydroxygruppe, welche nach beendeter Reaktion nach an sich bekannten Verfahren leicht wieder entfernt werden können, sind z. B. bekannt aus E. McOmie »Protective Groups in Organic Chemistry«, Plenum Press, London 1971, S. 95ff.

Die Umsetzung erfolgt zweckmäßigerweise in einem unter den Reaktionsbedingungen inerten Lösungsmittel bei Temperaturen zwischen 0°C und Siedetemperatur des Lösungsmittels. Im allgemeinen sind Temperaturen zwischen 0°C und 100°C vorteilhaft. Als Lösungsmittel eignen sich beispielsweise niedere Alkohole wie Methanol, Äthanol, Isopropanol, Butanol oder tert.-Butanol, aber auch aromatische Kohlenwasserstoffe wie Benzol oder Toluol, Dimethylformamid, Sulfolan, Hexamethylphosphortriamid, Tetramethylharnstoff oder cyclische Äther, wie z. B. Dioxan oder Tetrahydrofuran.

Als Alkalimetallsalze der 1-Phenyl-1,2-dihydro-3H-indazol-3-on-Verbindungen kommen Lithium-, Natrium- oder Kaliumsalze, vorzugsweise das Natriumsalz, in Frage, welche in situ durch Umsetzen der Verbindungen der Formel VI mit Alkalimetallalkoholaten oder -hydriden erhalten werden können. Bei der Alkylierung der 1-Phenyl-1,2-dihydro-3H-indazol-3-on-Verbindungen der Formel VI mit den Verbindungen der Formel VII werden im allgemeinen Gemische aus dem gewünschten N-alkylierten Produkt und dem dazu isomeren O-alkylierten Produkt erhalten. Aus diesen Gemischen kann das N-alkylierte Produkt chromatographisch oder durch Kristallisation abgetrennt werden.

Die O-alkylierten Nebenprodukte können durch einfaches Erhitzen in die entsprechenden N-alkylierten Produkte umgelagert werden. Die Umlagerungstemperatur liegt zweckmäßigerweise zwischen 60° und 200°C. Gewünschtenfalls kann die Umlagerung in Gegenwart eines inerten Lösungsmittels zweckmäßigerweise bei Siedetemperatur des Lösungsmittels durchgeführt werden. Geeignete Lösungsmittel sind z. B. in dem angegebenen Bereich siedende niedere Alkohole, beispielsweise Methanol, Butanol oder Isopentanol, oder aromatische Kohlenwasserstoffe wie Benzol, Toluol oder Xylol. Für die thermische Umlagerungsreaktion können auch die bei der Alkylierung anfallenden Gemische aus N-alkyliertem Produkt und dem dazu isomeren O-alkylierten Produkt direkt ohne vorherige Trennung eingesetzt werden.

Nach beendeter Reaktion der Verbindungen der Formel VI mit Verbindungen der Formel VII kann eine geschützte Hydroxygruppe nach an sich bekannten Verfahren durch Abspaltung der Schutzgruppe wieder freigesetzt werden und mit gebräuchlichen Halogenierungsmitteln, wie beispielsweise Thionylchlorid, Phosphoroxidchlorid oder Phosphortribromid, umgesetzt werden, um Verbindungen der Formel II a zu erhalten, worin Y Halogen bedeutet; oder die freigesetzte Hydroxygruppe kann nach an sich bekannten Methoden verestert werden, beispielsweise mit einem entsprechenden Säurehalogenid umgesetzt werden, um Verbindungen der Formel II a zu erhalten, worin Y einen reaktiven Esterrest, insbesondere einen der vorgenannten Sulfonsäurereste bedeutet.

Verbindungen der Formel III sind in der Literatur bisher noch nicht beschrieben worden und stellen neue wertvolle Zwischenprodukte für die Herstellung von pharmakologisch aktiven Verbindungen, beispielsweise den Verbindungen der Formel I, dar.

Verbindungen der Formel III können nach an sich bekannten Methoden erhalten werden, indem man beispielsweise Verbindungen der Formel II a mit einem Überschuß Piperazin umsetzt. Die Umsetzung kann nach an sich zur Alkylierung von Aminen üblichen Methoden, beispielsweise unter den vorstehend für die Umsetzung von Verbindungen der Formel II a mit Verbindungen der Formel V beschriebenen Bedingungen, durchgeführt werden.

Verbindungen der Formel III können auch aus Verbindungen der Formel VIII

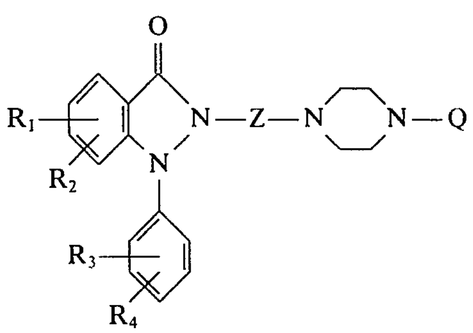

(VIII)

worin $R_1$, $R_2$, $R_3$, $R_4$ und Z obige Bedeutung besitzen und Q für eine Aminschutzgruppe steht, erhalten werden, indem man die Aminschutzgruppe auf an sich bekannte Weise abspaltet. Als Aminschutzgruppen kommen die an sich bekannten zum Schutz einer Aminofunktion gebräuchlichen Schutzgruppen, beispielsweise hydrolytisch abspaltbare Acylgruppen oder hydrogenolytisch abspaltbare Benzylgruppen in Frage. Geeignete Schutzgruppen sind z. B. bekannt aus E. McOmie »Protective Groups in Organic Chemistry«; Plenum Press, (1971) London S. 44ff. Insbesondere eignen sich die Formylgruppen und niedere Carbalkoxyschutzgruppen. Diese können in an sich bekannter Weise durch saure oder alkalische Hydrolyse abgespalten werden. Verbindungen der Formel VIII können auf an sich bekannte Weise erhalten werden, beispielsweise durch Umsetzung von Verbindungen der Formel II a mit Verbindungen der Formel IX

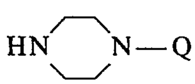

(IX)

worin Q obige Bedeutung besitzt. Die Umsetzung kann nach zur Alkylierung von Aminen üblichen Methoden, beispielsweise unter den für die Umsetzung von Verbindungen der Formel II a mit Verbindungen der Formel V beschriebenen Reaktionsbedingungen, erfolgen.

Die 1-Phenyl-1,2-dihydro-3 H-indazol-3-on-Verbindungen der allgemeinen Formel VI sind bekannt oder können nach an sich bekannten Methoden hergestellt werden (siehe z. B. Synthesis, 1978, 633–648).

Verbindungen der Formel V sind bekannt oder können nach an sich bekannten Methoden hergestellt werden, beispielsweise durch Umsetzung von Aminen der Formel X

$$H_2N—R_5 \qquad (X)$$

worin $R_5$ obige Bedeutung besitzt, mit entsprechenden Di(halogenalkyl)aminen unter zur Alkylierung von Aminen üblichen Bedingungen.

Gewünschtenfalls können in den Verbindungen der Formel I oder in den vorgenannten Zwischenprodukten einige der Substituenten in den Phenylringen oder dem Indazolgerüst auf an sich bekannte Weise nachträglich eingeführt werden bzw. gegen einen anderen Substituenten ausgetauscht werden. Sie können beispielsweise Halogen-Substituenten in das Indazol-System nach bekannten Methoden

nachträglich eingeführt werden. Man erhält mit Halogenierungsmitteln wie Chlor, Brom, N-Chlorsuccinimid, N-Chloracetamid oder N-Bromsuccinimid die entsprechenden halogenierten Verbindungen.

Die Verbindungen der Formel I und ihre pharmakologisch akzeptablen Säureadditionssalze zeichnen sich durch interessante pharmakologische Eigenschaften aus und zeigen insbesondere antiallergische Wirkungen. Außerdem besitzen die Verbindungen eine gute Verträglichkeit bei nur geringer Toxizität und zeigen insbesondere einen großen Abstand zwischen therapeutisch wirksamer und toxischer Dosis.

Aufgrund ihrer antiallergischen Wirkungen eignen sich die Verbindungen der Formel I und ihre pharmakologisch akzeptablen Salze als Antiallergika zur Behandlung allergischer Erkrankungen wie z. B. Bronchialasthma oder allergischer Rhinitis.

Die antiallergischen Eigenschaften der Verbindungen der Formel I können in pharmakologischen Standardtests an kleinen Tieren nachgewiesen werden. Beispielsweise weisen die Substanzen inhibierende Wirkungen gegenüber der zu allergischen Reaktionen führenden Freisetzung endogener Mediatoren aus den Mastzellen oder basophilen Leukozyten auf. Die zu verwendenden Dosen variieren naturgemäß je nach Art der verwendeten Substanz, der Applikationsart und des zu behandelnden Zustandes. Im allgemeinen werden jedoch befriedigende Ergebnisse in Tierversuchen mit Dosen zwischen 0,05 und 75 mg pro kg Körpergewicht p. o. erhalten. So zeigen die neuen Verbindungen eine spezifische hemmende Wirkung in dem nachstehend beschriebenen PCA-Test (Passive Cutane Anaphylaxie) an der Ratte.

Beschreibung der Testmethode zur Bestimmung der Hemmung der passiven cutanen Anaphylaxie (PCA-Test siehe Arch. int. pharmacology 252 (1981) 316–326).

Zur Herstellung des in dem Test verwendeten IgE-Antiovoalbumin-Serums nach der Methode von Mota (Immunology 7, (1964) 681) und J. Goose (Immunology 16 (1969) 749) wurden männliche Wistar-Ratten von 200–250 g Körpergewicht durch s. c. Injektion von 1 mg Ovoalbumin und 1 ml Bordetella-Pertussis-Suspension (Vaxicoq® Merieux $3 \cdot 10^{10}$ Organismen/ml) sensibilisiert. Nach 14 Tagen werden die Tiere entblutet und das Blut zentrifugiert. Das so erhaltene Antiserum wird bei 20°C gelagert.

An je vier verschiedenen Stellen des rasierten Rückens von nicht sensibilisierten Ratten werden je 0,1 ml Antiserum in die Haut injiziert. Nach 72 Stunden werden eine Lösung der Testverbindung oder zum Vergleich nur das Lösungsmittel oral appliziert und 10 Minuten später 5 mg Ovoalbumin und 5 mg blauer Farbstoff (Evans Blue) in 0,9%iger NaCl-Lösung i. p. appliziert. Nach 30 Minuten werden die Tiere getötet und die Durchmesser der an den mit Antiserum injizierten Stellen entstandenen blauen Flecken festgestellt. Der Hemmeffekt der Testsubstanz wird aus der Größe der auftretenden blauen Flecken bestimmt.

Die folgende Tabelle gibt nach dem vorstehend beschriebenen Test erhaltene Ergebnisse wieder. Die für die Verbindungen der Formel I angegebenen Beispielsnummern beziehen sich auf die nachstehenden Herstellungsbeispiele.

| Test Substanz der Formel I Beispiel Nr. | P. C. A. Hemmung $ED_{50}$, mg/kg |
|---|---|
| 12 | 22 |
| 18 | 25 |
| 17 | 17,9 |
| 19 | 11,5 |
| 15 | 10,6 |
| 20 | 7,5 |
| 21 | 19,2 |
| 49 | 15,5 |
| 32 | 6,6 |

Fortsetzung

| Test Substanz der Formel I Beispiel Nr. | P. C. A. Hemmung $ED_{50}$, mg/kg |
|---|---|
| 26 | 22 |
| 28 | 5,7 |
| 29 | 14 |

Bestimmung der minimalen toxischen Dosis an der Maus

Bei oraler Verabreichung der vorstehenden Substanzen in Dosen von bis zu 300 mg/kg konnten keine toxischen Symptome festgestellt werden.

Als Heilmittel können die Verbindungen der Formel I und ihre pharmakologisch verträglichen Salze zusammen mit üblichen pharmazeutischen Hilfsstoffen in galenischen Zubereitungen wie z. B. Tabletten, Kapseln, Suppositorien oder Lösungen enthalten sein. Diese galenischen Zubereitungen können nach an sich bekannten Methoden hergestellt werden unter Verwendung üblicher fester Trägerstoffe wie z. B. Talkum, Milchzucker oder Stärke oder flüssiger Verdünnungsmittel wie z. B. Wasser, fetten Ölen oder flüssigen Paraffinen.

Die Verbindungen der Formel I können in pharmazeutischen Gebrauchsformen verabreicht werden, welche etwa 0,5 bis 100 mg, vorzugsweise 0,5—25 mg Aktivsubstanz pro Einzeldosis enthalten. Die zu verwendende Dosierung wird selbstverständlich der zu behandelnden Spezies und den individuellen Erfordernissen angepaßt werden. Parenterale Formulierungen werden im allgemeinen weniger Aktivsubstanz enthalten als Präparate zur oralen Verabreichung.

Die nachfolgenden Beispiele sollen die Herstellung der neuen Verbindungen der Formel I sowie der neuen Zwischenprodukte näher erläutern jedoch den Umfang der Erfindung in keiner Weise beschränken.

Die Strukturen der neuen Verbindungen wurden durch spektroskopische Untersuchungen, insbesondere durch eine genaue Analyse der IR- und NMR-Spektren gesichert.

Die IR-Spektren der 1-Phenyl-1,2-dihydro-3 H-indazol-3-on-Verbindungen zeigen bei ca. 1700 $cm^{-1}$ die Carbonyl-Absorptionsbande des 1,2-Dihydro-3 H-indazol-3-on-Ringes und sind frei von C=N-Banden, die in 1 H-Indazolderivaten beobachtet werden können:

Beispiel 1

1-Phenyl-2-[4-(4-(2-methoxyphenyl)-piperazin-1-yl)-butyl]-1,2-dihydro-3 H-indazol-3-on.

A) 1-Phenyl-2-(4-brombutyl)-2,3-dihydro-1 H-indazol-3-on.

Zu einer Lösung von 3,2 g Natrium in 100 ml absol. Äthanol gibt man unter Rühren und Feuchtigkeitsausschluß eine Lösung von 26 g 1-Phenyl-1,2-dihydro-3 H-indazol-3-on in 270 ml absol. Äthanol. Man erwärmt 30 Min. auf 80°C, gibt nach Abkühlung 66 ml 1,4-Dibrombutan hinzu und erhitzt danach 3 Stunden unter Rückfluß. Anschließend wird das Äthanol im Vakuum abdestilliert, der Rückstand in Toluol aufgenommen, die Toluollösung mit verd. Natronlauge und Wasser gewaschen, über Natriumsulfat getrocknet, filtriert und im Vakuum eingedampft. Der Rückstand wird 30 Min. auf 190—210°C erhitzt und nach dem Abkühlen in Äther aufgenommen. Man erhält 19,1 g kristalline Substanz mit einem Schmelzpunkt von 102—106°C.

B) 19,1 g 1-Phenyl-2-(4-brombutyl)-1,2-dihydro-3 H-indazol-3-on werden in 360 ml Toluol mit 11,7 g N-(2-Methoxyphenyl)piperazin und 9 ml Triäthylamin 72 Stunden auf 100°C erhitzt. Nach dem Abkühlen wird die Reaktionslösung mit Wasser gewaschen und anschließend die organische Phase mit Salzsäure (20%) extrahiert. Die saure Phase wird einmal mit 50 ml Toluol extrahiert und dann mit Natronlauge (50%) unter Eiskühlung bis zur alkalischen Reaktion versetzt. Die ausgeschiedene Base wird in Methylenchlorid aufgenommen, mit Wasser gewaschen, über Natriumsulfat getrocknet, filtriert und vom Lösungsmittel befreit. Es werden 24,8 g Rohbase als Rückstand erhalten.

Der Rückstand wird in Äther gelöst, und die Lösung mit einer gesättigten Lösung von HCl-Gas in Äther versetzt. Dabei scheidet sich das Dihydrochlorid kristallin ab. Das Salz wird abfiltriert und aus Äthanol unter Zusatz von wenig Wasser umkristallisiert. Es werden 19,3 g Dihydrochlorid-Semihydrat der Titelverbindung erhalten.

Fp: 157—161°C.

Das Dihydrochlorid-Monohydrat mit 0,3 Mol Aceton hat einen Schmelzpunkt von 168—188°C.

## Beispiel 2

1-Phenyl-2-{3-[4-(4-trifluormethylphenyl)piperazin-1-yl]-propyl}-1,2-dihydro-3H-indazol-3-on

A) 1-Phenyl-2-(3-chlorpropyl)-1,2-dihydro-3H-indazol-3-on.

Zu einer Lösung des Natriumsalzes von 1-Phenyl-1,2-dihydro-3H-indazol-3-on (hergestellt aus 29,6 g 1-Phenyl-1,2-dihydro-3H-indazol-3-on und 3,6 g Natrium in 300 ml absol. Äthanol) gibt man 62 ml 1-Brom-3-chlorpropan und erhitzt 17 Std. unter Rückfluß. Das Äthanol wird im Vakuum abdestilliert, der Rückstand in Toluol aufgenommen und mit verd. Natronlauge und Wasser gewaschen. Nach dem Trocknen über Natriumsulfat wird die Lösung im Vakuum eingedampft. Das als Rückstand verbleibende rohe 1-Phenyl-2-[3-chlorpropyl]-1,2-dihydro-3H-indazol-3-on (40,5 g) wird an der zehnfachen Menge Kieselgel mit Toluol/Methylenchlorid/15% Äthanol chromatographiert.
Man erhält 15,9 g.

Fp: 69°C (Äthylacetat/Hexan).

B) 1-Phenyl-2-[3-(4-formylpiperazin-1-yl)-propyl]-1,2-dihydro-3H-indazol-3-on.

21,4 g 1-Phenyl-2-(3-chlorpropyl)-1,2-dihydro-3H-indazol-3-on werden mit 22,7 g N-Formylpiperazin und 2,1 g Kaliumbromid in 170 ml Isopropanol 18 Std. unter Rückfluß erhitzt. Danach wird das Isopropanol im Vakuum abdestilliert und der Rückstand in Toluol aufgenommen. Die Toluolphase wird mit verd. Salzsäure extrahiert, die salzsauren Extrakte mit verd. Natronlauge alkalisch gestellt und mit Methylenchlorid extrahiert. Nach dem Neutralwaschen der Methylenchloridphase wird über Natriumsulfat getrocknet und im Vakuum eingedampft.
Man erhält 17,4 g 1-Phenyl-2-[3-(4-formylpiperazin-1-yl)-propyl]-1,2-dihydro-3H-indazol-3-on.

C) 1-Phenyl-2-[3-(piperazin-1-yl)-propyl]-dihydro-3H-indazol-3-on.

17,4 g des wie unter 2 B beschrieben erhaltenen N-Formylpiperazinderivates werden in 150 ml eines 1 : 1-Gemisches aus Äthanol und 20%iger Salzsäure gelöst, über Nacht bei Raumtemperatur stehengelassen. Danach wird noch 2 Stunden am Rückfluß erhitzt und das Äthanol im Vakuum abdestilliert. Die als Rückstand verbleibende rohe Titelverbindung wird mit Toluol und verd. Natronlauge versetzt, die Toluolphase mit Wasser gewaschen, über Natriumsulfat getrocknet und im Vakuum eingedampft.
Man erhält 12,6 g Kristallisat mit einem Schmelzpunkt von 100—102°C.

D) 1-Phenyl-2-{3-[4-(4-trifluormethylphenyl)piperazin-1-yl]-propyl}-1,2-dihydro-3H-indazol-3-on.

13,6 g des unter 2 C beschriebenen Piperazinderivates werden in 100 ml Dimethylsulfoxid mit 10 g 4-Trifluormethylphenylbromid in Gegenwart von 10 g Kaliumkarbonat 16 Stunden auf 130°C erhitzt. Nach dem Abkühlen wird die Reaktionsmischung mit Wasser und verd. Salzsäure versetzt und mit Methylenchlorid extrahiert. Die salzsaure Phase wird mit verd. Natronlauge alkalisch gestellt und mit Toluol extrahiert. Die Toluolextrakte werden mit Wasser neutralgewaschen, über Natriumsulfat getrocknet und im Vakuum eingedampft. Man erhält 16 g 1-Phenyl-2-{3-[4-(4-trifluormethylphenyl)piperazin-1-yl]propyl}-1,2-dihydro-3H-indazol-3-on als Base.

Nach den in Beispiel 1—2 beschriebenen Verfahren können auch die in der folgenden Tabelle aufgeführten 1-Phenyl-2-[(piperazin-1-yl)-alkyl]-1,2-dihydro-3H-indazol-3-on-Verbindungen aus entsprechenden Verbindungen der Formel II oder III hergestellt werden.

| Bsp. Nr. | $R_1$ | $R_2$ | $R_3$ | $R_4$ | Z | $R_5$ | Salz | Fp °C |
|---|---|---|---|---|---|---|---|---|
| 3 | H | H | H | H | n-$C_3H_6$ | Phen | Base | öl |
| 4 | H | H | H | H | n-$C_3H_6$ | 2-Cl–Phen | Base | öl |
| 5 | H | H | H | H | n-$C_3H_6$ | 4-$CH_3O$–Phen | Base | öl |
| 6 | H | H | H | H | n-$C_3H_6$ | 3-$CF_3$–Phen | Base | öl |
| 7 | H | H | H | H | n-$C_3H_6$ | 4-Cl–Phen | Base | öl |
| 8 | H | H | H | H | n-$C_3H_6$ | 3-Cl–Phen | Base | öl |
| 9 | H | H | H | H | n-$C_4H_8$ | 3-Cl–Phen | Base | öl |
| 10 | H | H | H | H | n-$C_4H_8$ | 4-$CH_3O$–Phen | Base | öl |
| 11 | H | H | H | H | $C_2H_4$ | 4-$CH_3O$–Phen | Base | öl |
| 12 | H | H | H | H | n-$C_4H_8$ | Phen | HCl | 175–177 |
| 13 | H | H | H | H | n-$C_3H_6$ | 2-$CH_3O$–Phen | HCl | 130–195 |
| 14 | H | H | H | H | n-$C_5H_{10}$ | 4-F–Phen | HCl | 198–200 |
| 15 | H | H | H | H | n-$C_3H_6$ | 4-F–Phen | Base | öl |
| 16 | H | H | H | H | n-$C_4H_8$ | 2-Cl–Phen | HCl | 120–125 |
| 17 | H | H | H | H | n-$C_4H_8$ | 4-F–Phen | HCl | 157–160 |
| 18 | H | H | H | H | n-$C_4H_8$ | 2-Pyr | HCl | 210–212 |
| 19 | H | H | H | H | n-$C_3H_6$ | 2-Pyr | HCl | 185–190 |
| 20 | H | H | H | H | n-$C_4H_8$ | 4-$CH_3$–2-Pyr | HCl | 175–180 |
| 21 | H | H | H | H | n-$C_3H_6$ | 4-$CH_3$–2-Pyr | HCl | 169–170 |

Fortsetzung

| Bsp. Nr. | $R_1$ | $R_2$ | $R_3$ | $R_4$ | Z | $R_5$ | Salz | Fp °C |
|---|---|---|---|---|---|---|---|---|
| 22 | H | H | H | H | n-$C_4H_8$ | 2-$CH_3$—Phen | HCl | 195—200 |
| 23 | H | H | H | H | n-$C_4H_8$ | 2-Thien | Base | 110—115 (Z) |
| 24 | H | H | H | H | n-$C_4H_8$ | 3-Thien | Base | öl |
| 25 | 6-Cl | H | H | H | n-$C_4H_8$ | 4-$CH_3$—2-Pyr | Base | 110—112 |
| 26 | 6-Cl | H | H | H | n-$C_4H_8$ | 2-$CH_3O$—Phen | 2 HCl | 184—186 |
| 27 | 6-Cl | H | H | H | n-$C_4H_8$ | 4-F—Phen | HCl | 180—185 |
| 28 | 5-$CH_3O$ | H | H | H | n-$C_4H_8$ | 4-$CH_3$—2-Pyr | 2 HCl · 1 $H_2O$ | 180—185 |
| 29 | 5-$CH_3O$ | H | H | H | n-$C_4H_8$ | 2-$CH_3O$—Phen | Base | 85— 87 |
| 30 | 5-$CH_3$ | H | H | H | n-$C_3H_6$ | 2-Pyr | Base | 139—140 |
| 31 | H | H | 2,6-di-$CH_3$ | 2,6-di-$CH_3$ | n-$C_4H_8$ | Phen | 2 HCl · 1 $H_2O$ | 160—162 |
| 32 | H | H | 3-$CF_3$ | H | n-$C_4H_8$ | 4-$CH_3$—2-Pyr | 3 HCl | 150—155 |
| 33 | 5-$CH_3$ | H | 4-Cl | H | n-$C_4H_8$ | 2-$CH_3O$—Phen | 2 HCl | 158—160 |
| 34 | 5-$CH_3$ | H | 4-$CH_3O$ | H | n-$C_4H_8$ | 4-F—Phen | Base | öl |
| 35 | H | H | H | H | n-$C_4H_8$ | 3,4-O—$C_2H_4$—O—Phen | Base | öl |
| 36 | H | H | H | H | n-$C_4H_8$ | 3,4-O—$CH_2O$—Phen | Base | öl |
| 37 | H | H | H | H | n-$C_4H_8$ | 4-Pyr | Base | 155—157 |
| 38 | H | H | H | H | n-$C_4H_8$ | 3-Pyr | Base | öl |
| 39 | H | H | H | H | n-$C_4H_8$ | 2,6-di-$CH_3$—Phen | Base | öl |

Fortsetzung

| Bsp. Nr. | $R_1$ | $R_2$ | $R_3$ | $R_4$ | Z | $R_5$ | Salz | Fp °C |
|---|---|---|---|---|---|---|---|---|
| 40 | H | H | H | H | n-$C_4H_8$ | 3,4-di-$CH_3$—Phen | Base | öl |
| 41 | H | H | H | H | n-$C_4H_8$ | 4-OH—Phen | Base | öl |
| 42 | H | H | H | H | n-$C_4H_8$ | 4-$CH_3COO$—Phen | Base | öl |
| 43 | H | H | 3-$CF_3$ | H | n-$C_4H_8$ | 4-F—Phen | 2 HCl | 138–140 |
| 44 | H | H | H | H | n-$C_4H_8$ | 4-$CH_3O$—3-Pyr | Base | öl |
| 45 | H | H | H | H | n-$C_4H_8$ | 4-Cl—2-Pyr | Base | öl |
| 46 | 6,7-di-$CH_3O$ | 6,7-di-$CH_3O$ | H | H | n-$C_4H_8$ | 4-$CH_3$—2-Pyr | Base | öl |
| 47 | H | H | 4-$CH_3O$ | H | n-$C_4H_8$ | 4-F—Phen | Base | öl |
| 48 | 5-F | H | H | H | n-$C_4H_8$ | 4-$CH_3$—2-Pyr | Base | öl |
| 49 | H | H | H | H | n-$C_4H_8$ | 5-$CH_3$—2-Pyr | 3 HCl | 175–180 |

Phen = Phenyl  HCl = Hydrochlorid  Thien = Thienyl  Z = Zersetzung
Pyr = Pyridyl  Base = freie Base  öl = ölig

Beispiel I

Man stellt Tabletten in folgender Zusammensetzung her:

| | pro Tablette |
|---|---|
| 1-Phenyl-2-[4-(4-(2-methoxyphenyl)-piperazin-1-yl)-butyl]-1,2-dihydro-3 H-indazol-3-on | 25 mg |
| Maisstärke | 60 mg |
| Milchzucker | 130 mg |
| Gelatine (10% Lösung) | 6 mg |

Der Wirkstoff, die Maisstärke und der Milchzucker werden mit einer 10%igen Gelatine-Lösung eingedickt. Die Paste wird zerkleinert; das Granulat wird auf ein geeignetes Blech gebracht und bei 45°C getrocknet.
Das getrocknete Granulat wird durch eine Zerkleinerungsmaschine geleitet, in einem Mixer mit folgenden Ingredienzien vermischt:

| | |
|---|---|
| Talkum | 5 mg |
| Magnesiumstearat | 5 mg |
| Maisstärke | 9 mg |

und sodann zu Tabletten von 240 mg verpreßt.

**Patentansprüche für die Vertragsstaaten: BE, CH, DE, FR, GB, IT, LI, LU, NL, SE**

1. 1-Phenylindazol-3-on-Verbindungen der allgemeinen Formel I

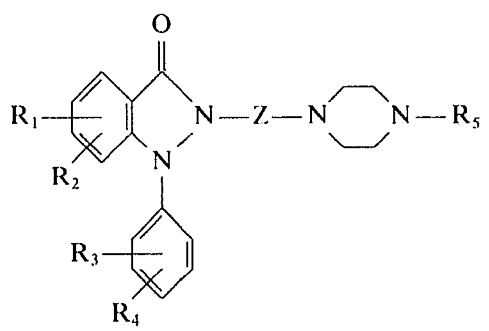

(I)

worin

$R_1$ Wasserstoff, Alkyl mit 1—4 Kohlenstoffatomen, Alkoxy mit 1—4 Kohlenstoffatomen, Halogen oder Trifluormethyl bedeutet,
$R_2$ Wasserstoff, Alkyl mit 1—4 Kohlenstoffatomen, Alkoxy mit 1—4 Kohlenstoffatomen oder Halogen bedeutet,
$R_3$ Wasserstoff, Alkyl mit 1—4 Kohlenstoffatomen, Alkoxy mit 1—4 Kohlenstoffatomen, Halogen oder Trifluormethyl bedeutet,
$R_4$ Wasserstoff, Alkyl mit 1—4 Kohlenstoffatomen, Alkoxy mit 1—4 Kohlenstoffatomen oder Halogen bedeutet,
Z eine Alkylengruppe mit 2—6 Kohlenstoffatomen bedeutet,
$R_5$ Pyridyl, welches unsubstituiert oder durch Alkyl mit 1—4 Kohlenstoffatomen, Halogen oder Alkoxy mit 1—4 Kohlenstoffatomen monosubstituiert ist, oder Thienyl oder eine gegebenenfalls substituierte Phenylgruppe a bedeutet,

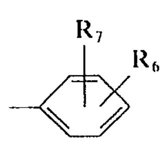

(a)

worin

$R_6$ Wasserstoff, Alkyl mit 1—4 Kohlenstoffatomen, Alkoxy mit 1—4 Kohlenstoffatomen, Hydroxy, Halogen, Trifluormethyl oder Alkanoyloxy mit 1—4 Kohlenstoffatomen bedeutet,

$R_7$ Wasserstoff, Alkyl mit 1—4 Kohlenstoffatomen, Alkoxy mit 1—4 Kohlenstoffatomen oder Halogen bedeutet, oder

$R_6$ und $R_7$ an benachbarte Kohlenstoffatome gebunden sind und zusammen Methylendioxy oder Äthylendioxy bedeuten,

sowie deren Säureadditionssalze.

2. 1-Phenylindazol-3-on-Verbindungen gemäß Anspruch 1, worin Z, $R_1$, $R_2$, $R_3$ und $R_4$ obige Bedeutung besitzen und $R_5$ für eine gegebenenfalls substituierte Phenylgruppe a steht

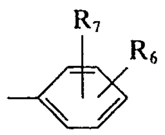

(a)

worin $R_6$ und $R_7$ obige Bedeutung besitzen.

3. 1-Phenylindazol-3-on-Verbindungen gemäß Anspruch 1, worin Z, $R_1$, $R_2$, $R_3$ und $R_4$ obige Bedeutung besitzen und $R_5$ Pyridyl, welches unsubstituiert oder durch Alkyl mit 1—4 Kohlenstoffatomen, Halogen oder Alkoxy mit 1—4 Kohlenstoffatomen monosubstituiert ist, bedeutet.

4. 1-Phenyl-2-{4-[4-(4-fluorphenyl)-piperazin-1-yl]-butyl}-1,2-dihydro-3H-indazol-3-on und dessen Säureadditionssalze gemäß Anspruch 2.

5. 1-Phenyl-2-{4-[4-(2-methoxyphenyl)-piperazin-1-yl]-butyl}-1,2-dihydro-3H-indazol-3-on und dessen Säureadditionssalze gemäß Anspruch 2.

6. 1-Phenyl-2-{4-[4-(2-pyridyl)-piperazin-1-yl]-butyl}-1,2-dihydro-3H-indazol-3-on und dessen Säureadditionssalze gemäß Anspruch 3.

7. 1-(4-Methoxyphenyl)-2-{4-[4-(4-fluorphenyl)-piperazin-1-yl]-butyl}-1,2-dihydro-3H-indazol-3-on und dessen Säureadditionssalze gemäß Anspruch 2.

8. Verbindungen der allgemeinen Formel II

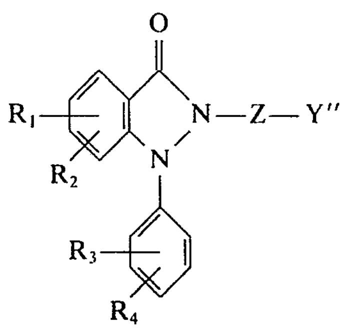

(II)

worin $R_1$, $R_2$, $R_3$, $R_4$ und Z die in Anspruch 1 angegebene Bedeutung besitzen und Y″ einen aminolytisch abspaltbaren Rest oder Hydroxy bedeutet, mit Ausnahme solcher Verbindungen, worin $R_1$ bis $R_4$ je Wasserstoff, Y″ Chlor und Z Äthylen oder Propylen bedeuten, und deren Säureadditionssalze.

9. Verbindungen gemäß Anspruch 8, worin $R_1$, $R_2$, $R_3$, $R_4$ und Z obige Bedeutung besitzen und Y″ Halogen, Hydroxy, Alkansulfonyloxy mit 1—4 Kohlenstoffatomen, Benzolsulfonyloxy oder im Benzolring durch Alkyl mit 1—4 Kohlenstoffatomen oder Halogen substituiertes Benzolsulfonyloxy bedeutet.

10. Verbindungen der allgemeinen Formel III

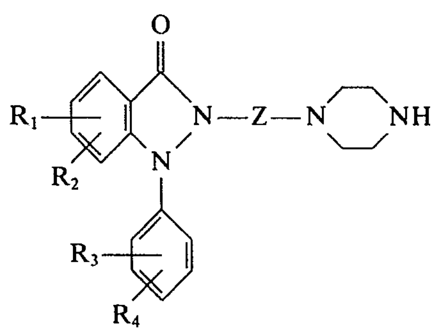

(III)

worin $R_1$, $R_2$, $R_3$, $R_4$ und Z die in Anspruch 1 angegebene Bedeutung besitzen, und deren Säureadditionssalze.

11. Verfahren zur Herstellung von 1-Phenylindazol-3-on-Verbindungen der allgemeinen Formel I

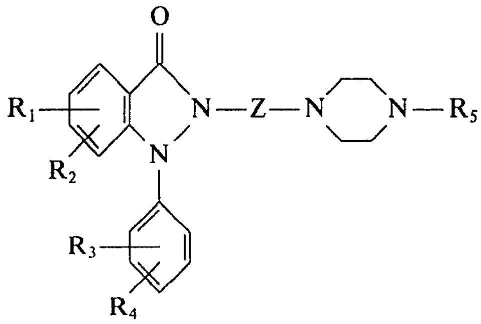

(I)

worin $R_1$, $R_2$, $R_3$, $R_4$, $R_5$ und Z die in Anspruch 1 angegebene Bedeutung besitzen, sowie deren Säureadditionssalzen, dadurch gekennzeichnet, daß man

a) Verbindungen der Formel II a

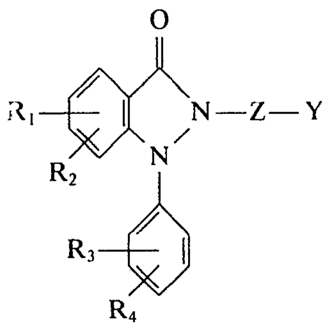

(II a)

worin $R_1$, $R_2$, $R_3$, $R_4$ und Z obige Bedeutung besitzen, und Y einen aminolytisch abspaltbaren Rest bedeutet, mit einer Verbindung der Formel V

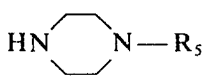

(V)

worin $R_5$ obige Bedeutung besitzt, umsetzt, oder

b) zur Herstellung von Verbindungen der Formel I a

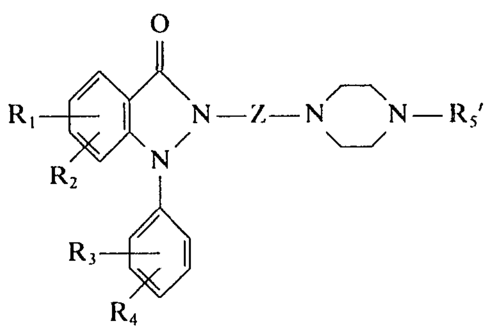

(I a)

worin Z, $R_1$, $R_2$, $R_3$ und $R_4$ obige Bedeutung besitzen und $R_5$ für eine substituierte Phenylgruppe a' steht

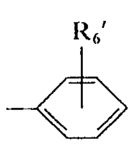

(a')

worin $R_6'$ ortho- oder paraständig ist und $CF_3$ bedeutet, Verbindungen der Formel III

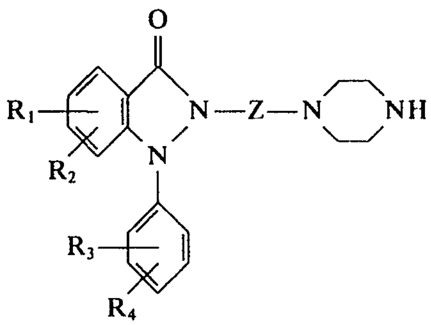

(III)

worin $R_1$, $R_2$, $R_3$, $R_4$ und Z obige Bedeutung besitzen, mit Verbindungen der Formel IV

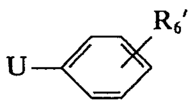

(IV)

worin $R_6{}'$ obige Bedeutung besitzt und U Halogen bedeutet, umsetzt, und gegebenenfalls freie Verbindungen der Formel I in ihre Säureadditionssalze überführt oder die Säureadditionssalze in die freien Verbindungen der Formel I überführt.

12. Arzneimittel, enthaltend eine pharmakologisch wirksame Menge einer 1-Phenylindazol-3-on-Verbindung gemäß Anspruch 1 und übliche pharmazeutische Hilfs- und/oder Trägerstoffe.

**Patentansprüche für den Vertragsstaat: AT**

1. Verfahren zur Herstellung von 1-Phenylindazol-3-on-Verbindungen der allgemeinen Formel I

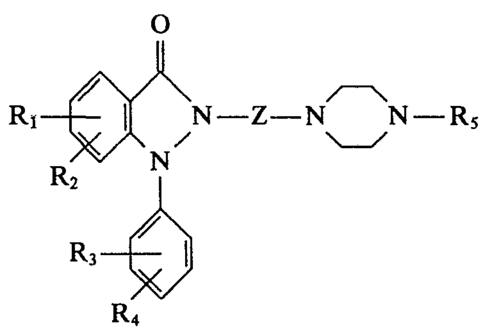

(I)

worin

$R_1$ Wasserstoff, Alkyl mit 1–4 Kohlenstoffatomen, Alkoxy mit 1–4 Kohlenstoffatomen, Halogen oder Trifluormethyl bedeutet,
$R_2$ Wasserstoff, Alkyl mit 1–4 Kohlenstoffatomen, Alkoxy mit 1–4 Kohlenstoffatomen oder Halogen bedeutet,
$R_3$ Wasserstoff, Alkyl mit 1–4 Kohlenstoffatomen, Alkoxy mit 1–4 Kohlenstoffatomen, Halogen oder Trifluormethyl bedeutet,
$R_4$ Wasserstoff, Alkyl mit 1–4 Kohlenstoffatomen, Alkoxy mit 1–4 Kohlenstoffatomen oder Halogen bedeutet,
Z eine Alkylengruppe mit 2–6 Kohlenstoffatomen bedeutet,
$R_5$ Pyridyl, welches unsubstituiert oder durch Alkyl mit 1–4 Kohlenstoffatomen, Halogen oder Alkoxy mit 1–4 Kohlenstoffatomen monosubstituiert ist, Thienyl oder eine gegebenenfalls substituierte Phenylgruppe a bedeutet

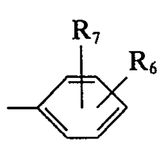

(a)

worin

$R_6$ Wasserstoff, Alkyl mit 1—4 Kohlenstoffatomen, Alkoxy mit 1—4 Kohlenstoffatomen, Hydroxy, Halogen, Trifluormethyl oder Alkanoyloxy mit 1—4 Kohlenstoffatomen bedeutet,
$R_7$ Wasserstoff, Alkyl mit 1—4 Kohlenstoffatomen, Alkoxy mit 1—4 Kohlenstoffatomen oder Halogen bedeutet, oder
$R_6$ und $R_7$ an benachbarte Kohlenstoffatome gebunden sind und zusammen Methylendioxy oder Äthylendioxy bedeuten,

sowie deren Säureadditionssalzen, dadurch gekennzeichnet, daß man

a) Verbindungen der Formel II a

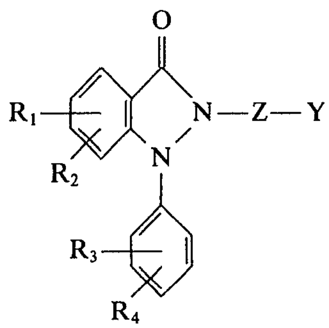

(IIa)

worin $R_1$, $R_2$, $R_3$, $R_4$ und Z obige Bedeutung besitzen, und Y einen aminolytisch abspaltbaren Rest bedeutet, mit einer Verbindung der Formel V

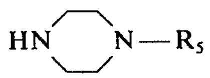

(V)

worin $R_5$ obige Bedeutung besitzt, umsetzt, oder
b) zur Herstellung von Verbindungen der Formel I a

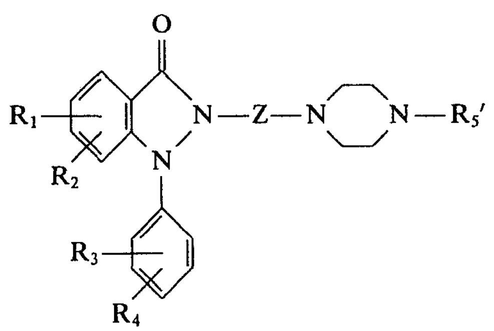

(Ia)

worin Z, $R_1$, $R_2$, $R_3$ und $R_4$ obige Bedeutung besitzen und $R_5'$ für eine substituierte Phenylgruppe a' steht

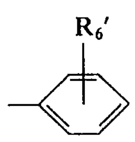

(a')

worin $R_6'$ ortho- oder paraständig ist und $CF_3$ bedeutet, Verbindungen der Formel III

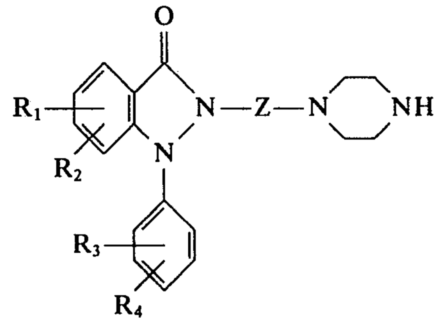

(III)

worin $R_1$, $R_2$, $R_3$, $R_4$ und Z obige Bedeutung besitzen, mit Verbindungen der Formel IV

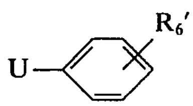

(IV)

worin $R_6'$ obige Bedeutung besitzt und U Halogen bedeutet, umsetzt, und gegebenenfalls freie Verbindungen der Formel I in ihre Säureadditionssalze überführt oder die Säureadditionssalze in die freien Verbindungen der Formel I überführt.

2. Verfahren nach Anspruch 1, dadurch gekennzeichnet, daß man als Verbindungen der Formel I 1-Phenylindazol-3-on-Verbindungen herstellt, worin Z, $R_1$, $R_2$, $R_3$ und $R_4$ obige Bedeutung besitzen und $R_5$ für eine gegebenenfalls substituierte Phenylgruppe a steht

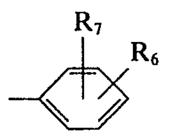

(a)

worin $R_6$ und $R_7$ obige Bedeutung besitzen.

3. Verfahren nach Anspruch 1, dadurch gekennzeichnet, daß man als Verbindungen der Formel I 1-Phenylindazol-3-on-Verbindungen gemäß Anspruch 1, herstellt, worin Z, $R_1$, $R_2$, $R_3$ und $R_4$ obige Bedeutung besitzen und $R_5$ Pyridyl, welches unsubstituiert oder durch Alkyl mit 1—4 Kohlenstoffatomen, Halogen oder Alkoxy mit 1—4 Kohlenstoffatomen monosubstituiert ist, bedeutet.

4. Verfahren nach Anspruch 2, dadurch gekennzeichnet, daß man als Verbindungen der Formel I 1-Phenyl-2-{4-[4-(4-fluorphenyl)-piperazin-1-yl]-butyl}-1,2-dihydro-3 H-indazol-3-on und dessen Säureadditionssalze herstellt.

5. Verfahren nach Anspruch 2, dadurch gekennzeichnet, daß man als Verbindungen der Formel I 1-Phenyl-2-{4-[4-(2-methoxyphenyl)-piperazin-1-yl]-butyl}-1,2-dihydro-3 H-indazol-3-on und dessen Säureadditionssalze herstellt.

6. Verfahren nach Anspruch 3, dadurch gekennzeichnet, daß man als Verbindungen der Formel I 1-Phenyl-2-{4-[4-(2-pyridyl)-piperazin-1-yl]-butyl}-1,2-dihydro-3 H-indazol-3-on und dessen Säureadditionssalze herstellt.

7. Verfahren nach Anspruch 2, dadurch gekennzeichnet, daß man als Verbindungen der Formel I 1-(4-Methoxyphenyl)-2-{4-[4-(4-fluorphenyl)-piperazin-1-yl]-butyl}-1,2-dihydro-3 H-indazol-3-on und dessen Säureadditionssalze herstellt.

8. Verfahren zur Herstellung von Verbindungen der allgemeinen Formel II

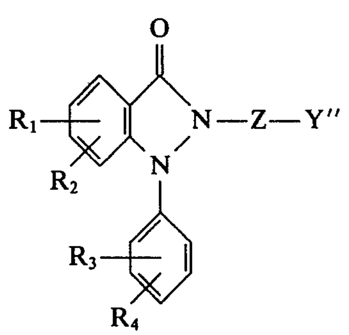

(II)

worin $R_1$, $R_2$, $R_3$, $R_4$ und Z die in Anspruch 1 angegebene Bedeutung besitzen und Y'' einen aminolytisch abspaltbaren Rest oder Hydroxy bedeutet, mit Ausnahme solcher Verbindungen, worin $R_1$ bis $R_4$ je Wasserstoff, Y'' Chlor und Z Äthylen oder Propylen bedeuten, und deren Säureadditionssalzen, dadurch

gekennzeichnet, daß man Alkalimetallsalze von 1-Phenyl-1,2-dihydro-3 H-indazol-3-on-Verbindungen der allgemeinen Formel VI

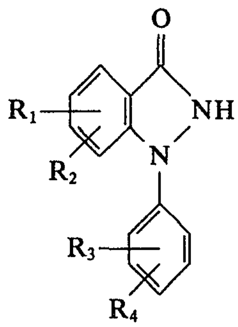

(VI)

worin $R_1$, $R_2$, $R_3$ und $R_4$ obige Bedeutung besitzen, mit Verbindungen der allgemeinen Formel VII

$$Y—Z—Y' \quad (VII)$$

worin Y einen aminolytisch abspaltbaren Rest und Y' einen aminolytisch abspaltbaren Rest oder eine geschützte Hydroxygruppe bedeuten, umsetzt und anschließend eine allfällige geschützte Hydroxygruppe durch Abspaltung der Schutzgruppe freisetzt.

9. Verfahren nach Anspruch 8, dadurch gekennzeichnet, daß man als Verbindungen der Formel II Verbindungen herstellt, worin $R_1$, $R_2$, $R_3$, $R_4$ und Z obige Bedeutung besitzen und Y'' Halogen, Hydroxy, Alkansulfonyloxy mit 1—4 Kohlenstoffatomen, Benzolsulfonyloxy oder im Benzolring durch Alkyl mit 1—4 Kohlenstoffatomen oder Halogen substituiertes Benzolsulfonyloxy bedeutet.

10. Verfahren zur Herstellung von Verbindungen der allgemeinen Formel III

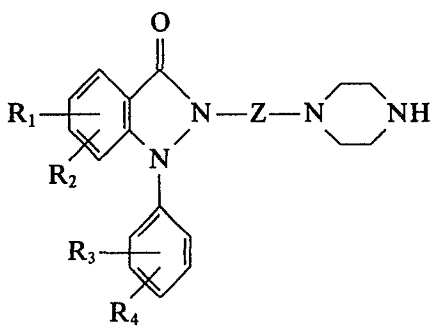

(III)

worin $R_1$, $R_2$, $R_3$, $R_4$ und Z die in Anspruch 1 angegebene Bedeutung besitzen und deren Säureadditionssalzen, dadurch gekennzeichnet, daß man Verbindungen der allgemeinen Formel II a

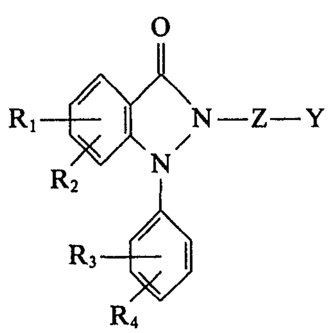

(II a)

worin $R_1$, $R_2$, $R_3$, $R_4$ und Z obige Bedeutung besitzen und Y einen aminolytisch abspaltbaren Rest bedeutet mit einem Überschuß Piperazin umsetzt oder aus Verbindungen der allgemeinen Formel VIII

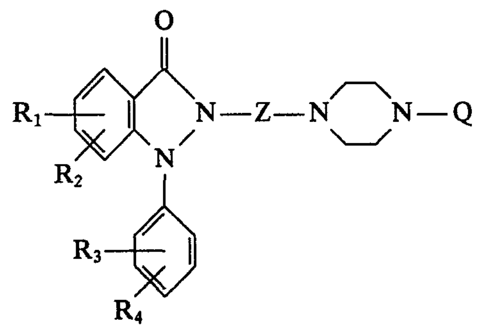

(VIII)

worin $R_1$, $R_2$, $R_3$, $R_4$ und Z obige Bedeutung besitzen und Q für eine Aminoschutzgruppe steht, diese Aminoschutzgruppe abspaltet.

**Claims for the contracting States: BE, CH, DE, FR, GB, IT, LI, LU, NL, SE**

1. 1-Phenylindazol-3-one compounds of the general formula I

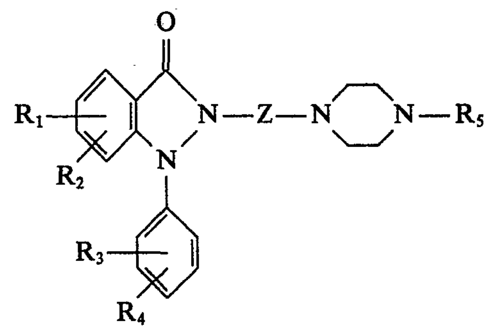

(I)

in which

$R_1$ is hydrogen, alkyl with 1 to 4 carbon atoms, alkoxy with 1 to 4 carbon atoms, halogen or trifluoromethyl,
$R_2$ is hydrogen, alkyl with 1 to 4 carbon atoms, alkoxy with 1 to 4 carbon atoms or halogen,
$R_3$ is hydrogen, alkyl with 1 to 4 carbon atoms, alkoxy with 1 to 4 carbon atoms, halogen or trifluoromethyl,
$R_4$ is hydrogen, alkyl with 1 to 4 carbon atoms, alkoxy with 1 to 4 carbon atoms or halogen,
Z is an alkylene group with 2 to 6 carbon atoms,
$R_5$ is pyridyl, which is unsubstituted or monosubstituted by alkyl with 1 to 4 carbon atoms, halogen or alkoxy with 1 to 4 carbon atoms, thienyl or an optionally substituted phenyl group a

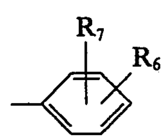

(a)

in which

$R_6$ is hydrogen, alkyl with 1 to 4 carbon atoms, alkoxy with 1 to 4 carbon atoms, hydroxy, halogen, trifluoromethyl or alkanoyloxy with 1 to 4 carbon atoms,
$R_7$ is hydrogen, alkyl with 1 to 4 carbon atoms, alkoxy with 1 to 4 carbon atoms or halogen or
$R_6$ and $R_7$ are linked to adjacent carbon atoms, and together form methylendioxy or ethylenedioxy,

and their acid addition salts.

2. 1-Phenylindazol-3-one compounds according to Claim 1, in which Z, $R_1$, $R_2$, $R_3$ and $R_4$ have the above meanings and $R_5$ stands for an optionally substituted phenyl group a

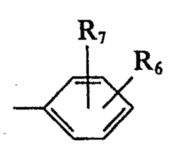

(a)

in which $R_6$ and $R_7$ have the above meanings.

3. 1-Phenylindazol-3-one compounds according to Claim 1 in which Z, $R_1$, $R_2$, $R_3$ and $R_4$ have the above meanings and $R_5$ is pyridyl which is unsubstituted or is mono-substituted by alkyl with 1 to 4 carbon atoms, halogen or alkoxy with 1 to 4 carbon atoms.

4. 1-Phenyl-2-{4-[4-(4-fluorophenyl)-piperazin-1-yl]-butyl}-1,2-dihyro-3 H-indazol-3-one and its acid addition salts according to Claim 2.

5. 1-Phenyl-2-{4-[4-(2-methoxyphenyl)-piperazin-1-yl]-butyl}-1,2-dihydro-3 H-indazol-3-one and its acid addition salts according to Claim 2.

6. 1-Phenyl-2-{4-[4-(2-pyridyl)-piperazin-1-yl]-butyl}-1,2-dihydro-3 H-indazol-3-one and its acid addition salts according to Claim 3.

7. 1-(4-Methoxyphenyl)-2-{4-[4-(4-fluorophenyl)-piperazin-1-yl]-butyl}-1,2-dihydro-3 H-indazol-3-one and its acid addition salts according to Claim 2.

8. Compounds of the general formula II

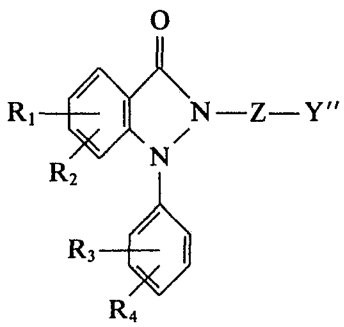

(II)

in which $R_1$, $R_2$, $R_3$, $R_4$ and Z have the meanings indicated in Claim 1 and Y″ is a radical which can be split off aminolytically or hydroxy, with the exception of those compounds in which each of $R_1$ to $R_4$ is hydrogen, Y″ is chlorine and Z is ethylene or propylene, and their acid addition salts.

9. Compounds according to Claim 8 in which $R_1$, $R_2$, $R_3$, $R_4$ and Z have the above meanings and Y″ is halogen, hydroxy, alkane-sulphonyloxy with 1 to 4 carbon atoms, benzene-sulphonyloxy or benzene-sulphonyloxy substituted in the benzene ring by alkyl with 1 to 4 carbon atoms or halogen.

10. Compounds of the general formula III

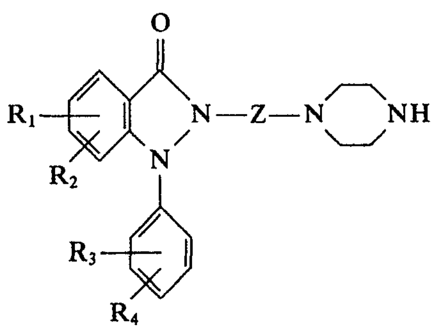

(III)

in which $R_1$, $R_2$, $R_3$, $R_4$ and Z have the meanings indicated in Claim 1, and their acid addition salts.

11. Process for the preparation of 1-phenylindazol-3-one compounds of the general formula I

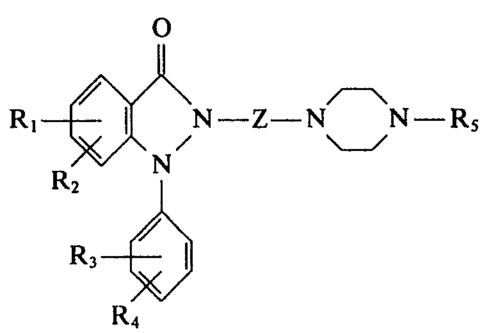

(I)

in which $R_1$, $R_2$, $R_3$, $R_4$, $R_5$ and Z have the meanings indicated in Claim 1, and their acid addition salts, characterized in that

a) compounds of formula II a

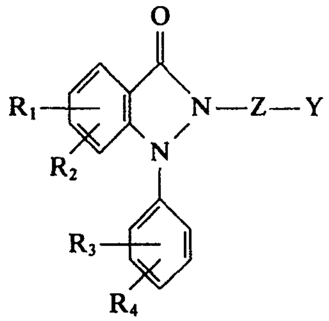

(II a)

in which $R_1$, $R_2$, $R_3$, $R_4$ and Z have the above meanings and Y is a radical which can be split off aminolytically, are reacted with a compound of formula V

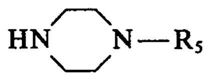

(V)

in which $R_5$ has the above meaning, or

b) for the preparation of compounds of formula I a

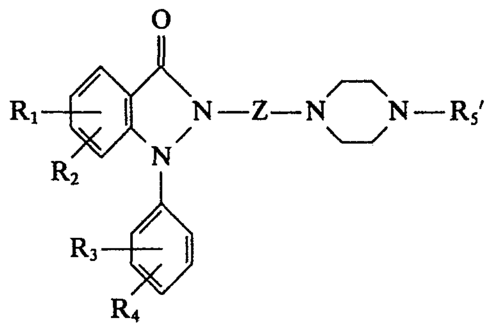

(I a)

in which Z, $R_1$, $R_2$, $R_3$ and $R_4$ have the above meanings and $R_5'$ stands for a substituted phenyl group a'

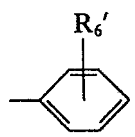

(a')

in which $R_6'$ is in the ortho or para position and is $CF_3$, compounds of formula III

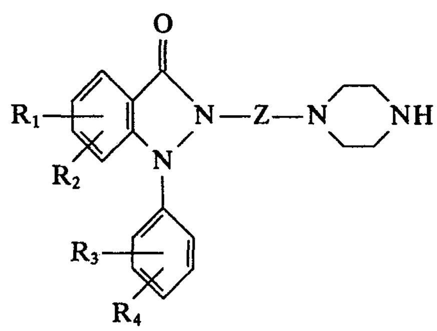

(III)

in which $R_1$, $R_2$, $R_3$, $R_4$ and Z have the above meanings, are reacted with compounds of formula IV

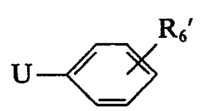

(IV)

in which $R_6'$ has the above meaning and U is halogen, and optionally free compounds of formula I

are converted into their acid addition salts or the acid addition salts are converted into the free compounds of formula I.

12. Medicaments containing a pharmacologically effective quantity of a 1-phenylindazol-3-one compound according to Claim 1 and conventional pharmaceutical adjuvant and/or carrier substances.

**Claims for the contracting State: AT**

1. Process for the preparation of 1-phenylindazol-3-one compounds of the general formula I

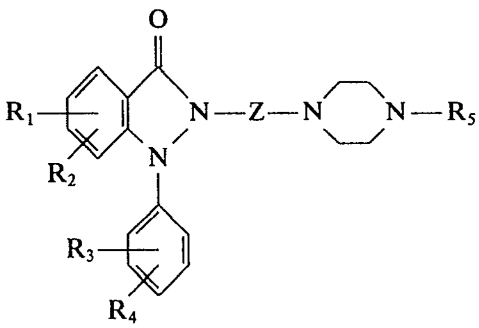

(I)

in which

$R_1$ is hydrogen, alkyl with 1 to 4 carbon atoms, alkoxy with 1 to 4 carbon atoms, halogen or trifluoromethyl,
$R_2$ is hydrogen, alkyl with 1 to 4 carbon atoms, alkoxy with 1 to 4 carbon atoms or halogen,
$R_3$ is hydrogen, alkyl with 1 to 4 carbon atoms, alkoxy with 1 to 4 carbon atoms, halogen or trifluoromethyl,
$R_4$ is hydrogen, alkyl with 1 to 4 carbon atoms, alkoxy with 1 to 4 carbon atoms or halogen,
Z is an alkylene group with 2 to 6 carbon atoms,
$R_5$ is pyridyl, which is unsubstituted or monosubstituted by alkyl with 1 to 4 carbon atoms, halogen or alkoxy with 1 to 4 carbon atoms, thienyl or an optionally substituted phenyl group a

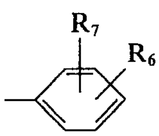

(a)

in which

$R_6$ is hydrogen, alkyl with 1 to 4 carbon atoms, alkoxy with 1 to 4 carbon atoms, hydroxy, halogen, trifluoromethyl or alkanoyloxy with 1 to 4 carbon atoms,
$R_7$ is hydrogen, alkyl with 1 to 4 carbon atoms, alkoxy with 1 to 4 carbon atoms or halogen or
$R_6$ and $R_7$ are linked to adjacent carbon atoms, and together form methylenedioxy or ethylenedioxy,

and their acid addition salts, characterized in that

a) compounds of formula II a

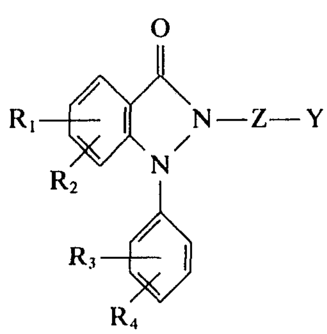

(II a)

in which $R_1$, $R_2$, $R_3$, $R_4$ and Z have the above meanings and Y is a radical which can be split off aminolytically, are reacted with a compound of formula V

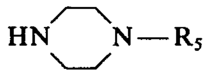

(V)

in which $R_5$ has the above meaning, or

b) for the preparation of compounds of formula I a

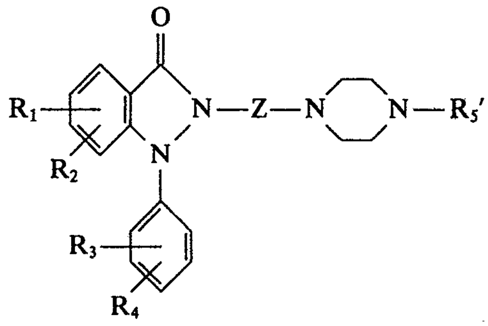

(Ia)

in which Z, $R_1$, $R_2$, $R_3$ and $R_4$ have the above meanings and $R_5'$ stands for a substituted phenyl group a'

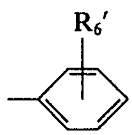

(a')

in which $R_6'$ is in the ortho or para position and is $CF_3$, compounds of formula III

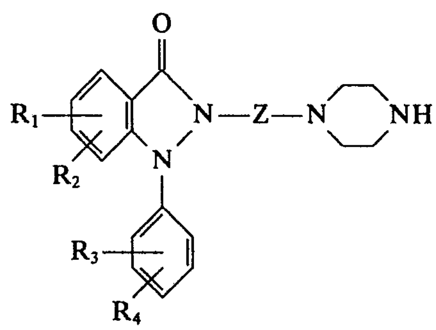

(III)

in which $R_1$, $R_2$, $R_3$, $R_4$ and Z have the above meanings, are reacted with compounds of formula IV

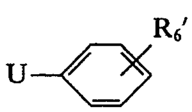

(IV)

in which $R_6'$ has the above meaning and U is halogen, and optionally free compounds of formula I are converted into their acid addition salts or the acid addition salts are converted into the free compounds of formula I.

2. Process according to Claim 1, characterized in that as compounds of formula I 1-phenylindazol-3-one compounds are prepared, in which Z, $R_1$, $R_2$, $R_3$ and $R_4$ have the above meanings and $R_5$ stands for an optionally substituted phenyl group a

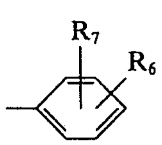

(a)

in which $R_6$ and $R_7$ have the above meanings.

3. Process according to Claim 1, characterized in that as compounds of formula I 1-phenylindazol-3-one compounds according to Claim 1 are prepared in which Z, $R_1$, $R_2$, $R_3$ and $R_4$ have the above meanings and $R_5$ is pyridyl which is unsubstituted or is monosubstituted by alkyl with 1 to 4 carbon atoms, halogen or alkoxy with 1 to 4 carbon atoms.

4. Process according to Claim 2, characterized in that as compounds of formula I, 1-phenyl-2-{4-[4-(4-fluorophenyl)-piperazin-1-yl]-butyl}-1,2-dihydro-3 H-indazol-3-one and its acid addition salts are prepared.

5. Process according to Claim 2, characterized in that as compounds of formula I, 1-phenyl-2-{4-[4-(2-methoxyphenyl)-piperazin-1-yl]-butyl}-1,2-dihydro-3 H-indazol-3-one and its acid addition salts are prepared.

6. Process according to Claim 3, characterized in that as compounds of formula I, 1-phenyl-2-{4-[4-(2-pyridyl)-piperazin-1-yl]-butyl}-1,2-dihydro-3 H-indazol-3-one and its acid addition salts are prepared.

7. Process according to Claim 2, characterized in that as compounds of formula I, 1-(4-methoxyphenyl)-2-{4-[4-(4-fluorophenyl)-piperazin-1-yl]-butyl}-1,2-dihydro-3 H-indazol-3-one and its acid addition salts are prepared.

8. Process for the preparation of compounds of the general formula II

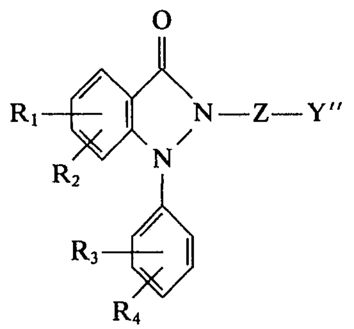

(II)

in which $R_1$, $R_2$, $R_3$, $R_4$ and Z have the meanings indicated in Claim 1 and Y″ is a radical which can be split off aminolytically or hydroxy, with the exception of those compounds in which each of $R_1$ to $R_4$ is hydrogen, Y″ is chlorine and Z is ethylene or propylene, and their acid addition salts, characterized in that alkali metal salts of 1-phenyl-1,3-dihydro-3 H-indazol-3-one compounds of the general formula VI

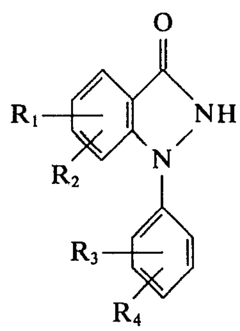

(VI)

in which $R_1$, $R_2$, $R_3$ and $R_4$ have the above meanings, are reacted with compounds of the general formula VII

$$Y—Z—Y' \quad \text{(VII)}$$

in which Y is a radical which can be split off aminolytically and Y′ is a radical which can be split off aminolytically or a protected hydroxy group, and subsequently any a protected hydroxy group is set free by splitting off the protecting group.

9. Process according to Claim 8, characterized in that as compounds of formula II, compounds are prepared in which $R_1$, $R_2$, $R_3$, $R_4$ and Z have the above meanings and Y″ is halogen, hydroxy, alkanesulphonyloxy with 1 to 4 carbon atoms, benzene-sulphonyloxy or benzene-sulphonyloxy substituted in the benzene ring by alkyl with 1 to 4 carbon atoms or halogen.

10. Process for the preparation of compounds of the general formula III

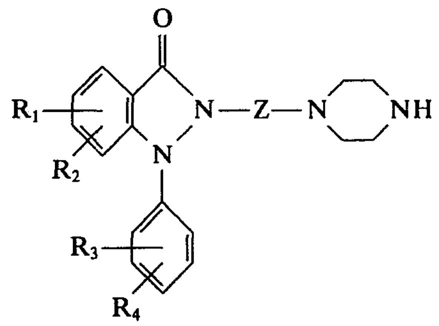

(III)

in which $R_1$, $R_2$, $R_3$, $R_4$ and Z have the meanings indicated in Claim 1, and their acid addition salts, characterized in that compounds of the general formula II a

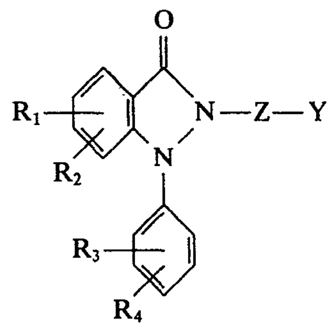

(II a)

in which $R_1$, $R_2$, $R_3$, $R_4$ and Z have the above meanings and Y is a radical which can be split off aminolytically, are reacted with a surplus of piperazine, or from compounds of the general formula VIII

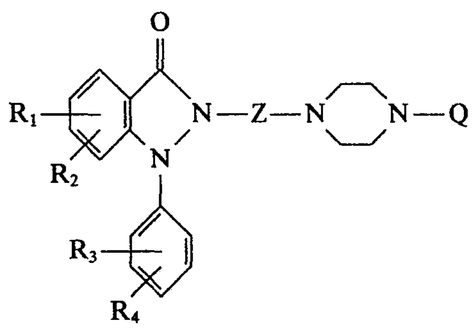

(VIII)

in which $R_1$, $R_2$, $R_3$, $R_4$ and Z have the above meanings and Q stands for an amino protecting group, this amino protecting group is split off.

**Revendications pour les Etats contractants: BE, CH, DE, FR, GB, IT, LI, LU, NL, SE**

1. Composés de 1-phénylindazol-3-one de formule générale I

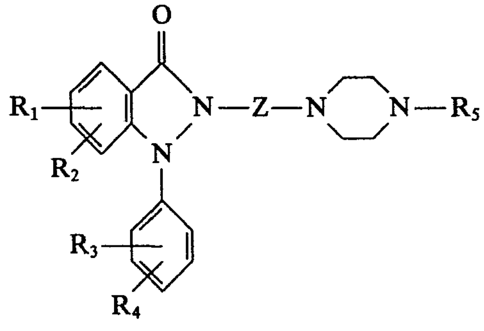

(I)

dans laquelle

$R_1$ représente de l'hydrogène, un alkyle avec 1 à 4 atomes de carbone, un alcoxy avec 1 à 4 atomes de carbone, un halogène ou un trifluorométhyle,
$R_2$ représente de l'hydrogène, un alkyle avec 1 à 4 atomes de carbone, un alcoxy avec 1 à 4 atomes de carbone ou un halogène,
$R_3$ représente de l'hydrogène, un alkyle avec 1 à 4 atomes de carbone, un alcoxy avec 1 à 4 atomes de carbone, un halogène ou un trifluorométhyle,
$R_4$ représente de l'hydrogène, un alkyle avec 1 à 4 atomes de carbone, un alcoxy avec 1 à 4 atomes de carbone ou un halogène,
Z représente un groupe alkylène avec 2 à 6 atomes de carbone,
$R_5$ représente un pyridyle qui est non substitué ou monosubstitué par un alkyle avec 1 à 4 atomes de carbone, un halogène ou un alcoxy avec 1 à 4 atomes de carbone, un thiényle ou un groupe phényle a éventuellement substitué

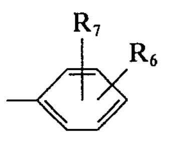

(a)

où

$R_6$ représente de l'hydrogène, un alkyle avec 1 à 4 atomes de carbone, un alcoxy avec 1 à 4 atomes de carbone, un hydroxy, un halogène, un trifluorométhyle ou un alcanoyloxy avec 1 à 4 atomes de carbone,
$R_7$ représente de l'hydrogène, un alkyle avec 1 à 4 atomes de carbone, un alcoxy avec 1 à 4 atomes de carbone ou un halogène ou
$R_6$ et $R_7$ sont fixés sur des atomes de carbone voisins et représentent ensemble un méthylènedioxy ou un éthylènedioxy,

ainsi que leurs sels d'addition avec des acides.

2. Composés de 1-phénylindazol-3-one selon la revendication 1, dans lesquels Z, $R_1$, $R_2$, $R_3$ et $R_4$ ont la signification ci-dessus et $R_5$ est un groupe phényle a éventuellement substitué

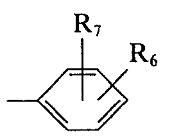

(a)

où $R_6$ et $R_7$ ont la signification précédente.

3. Composés de 1-phénylindazol-3-one selon la revendication 1, dans lesquels Z, $R_1$, $R_2$, $R_3$ et $R_4$ ont la signification ci-dessus et $R_5$ représente un pyridyle qui est non substitué ou monosubstitué par un alkyle avec 1 à 4 atomes de carbone, un halogène ou un alcoxy avec 1 à 4 atomes de carbone.

4. La 1-phényl-2-(4-[4-(4-fluorophényl)-pipérazin-1-yl]-butyl)-1,2-dihydro-3 H-indazol-3-one et ses sels d'addition avec des acides selon la revendication 2.

5. La 1-phényl-2-(4-[4-(2-méthoxyphényl)-pipérazin-1-yl]-butyl)-1,2-dihydro-3 H-indazol-3-one et ses sels d'addition avec des acides selon la revendication 2.

6. La 1-phényl-2-(4-[4-(2-pyridyl)-pipérazin-1-yl]-butyl)-1,2-dihydro-3 H-indazol-3-one et ses sels d'addition avec des acides selon la revendication 3.

7. La 1-(4-méthoxyphényl)-2-(4-[4-(4-fluorophényl)-pipérazin-1-yl]-butyl)-1,2-dihydro-3 H-indazol-3-one et ses sels d'addition avec des acides selon la revendication 2.

8. Composés de formule générale II

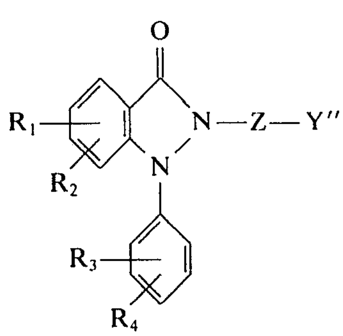

(II)

dans laquelle $R_1$, $R_2$, $R_3$, $R_4$ et Z ont la signification indiquée dans la revendication 1 et Y'' représente un reste éliminable par aminolyse ou un hydroxy, à l'exception des composés où $R_1$ à $R_4$ représentent chacun de l'hydrogène, Y'' représente du chlore et Z un éthylène ou un propylène et leurs sels d'addition avec des acides.

9. Composés selon la revendication 8, caractérisés en ce que $R_1$, $R_2$, $R_3$, $R_4$ et Z ont la signification cidessus et Y'' représente un halogène, un hydroxy, un alcanesulfonyloxy avec 1 à 4 atomes de carbone, un benzènesulfonyloxy ou un benzènesulfonyloxy substitué dans le noyau benzénique par un alkyle avec 1 à 4 atomes de carbone ou un halogène.

10. Composés de formule générale III

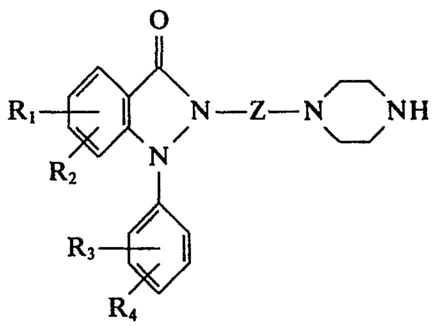

(III)

dans laquelle $R_1$, $R_2$, $R_3$, $R_4$ et Z ont la signification indiquée dans la revendication 1 et leurs sels d'addition avec des acides.

11. Procédé pour la préparation de composés de 1-phényl-3-one de formule générale I

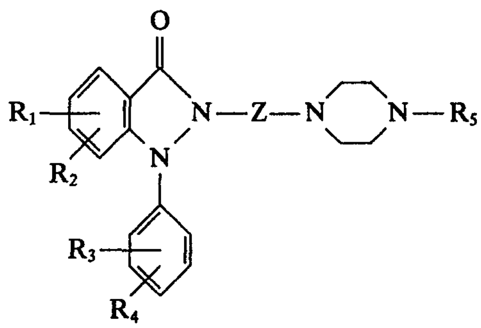

(I)

dans laquelle $R_1$, $R_2$, $R_3$, $R_4$, $R_5$ et Z ont la signification indiquée dans la revendication 1, ainsi que de leurs sels d'addition avec des acides, caractérisé en ce que

a) on fait réagir des composés de formule II a

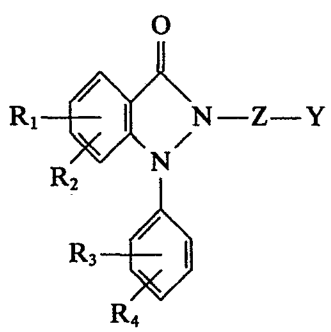

(II a)

dans laquelle $R_1$, $R_2$, $R_3$, $R_4$ et Z ont la signification indiquée plus haut et Y représente un reste éliminable par aminolyse, avec un composé de formule V

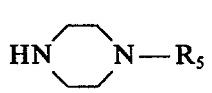

(V)

dans laquelle $R_5$ a la signification indiquée plus haut, ou que,

b) pour la préparation de composés de formule I a

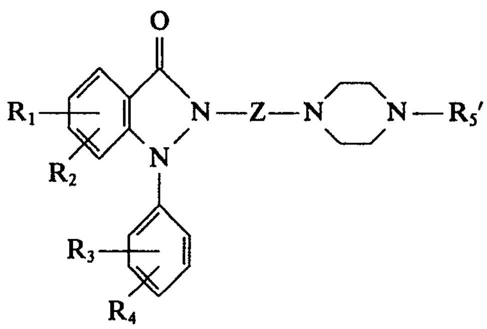

(Ia)

dans laquelle Z, $R_1$, $R_2$, $R_3$ et $R_4$ ont la signification ci-dessus et $R_5'$ est un reste phényle a' substitué

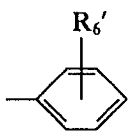

(a')

où $R_6'$ est placé en position ortho ou para et représente $CF_3$, on fait réagir des composés de formule III

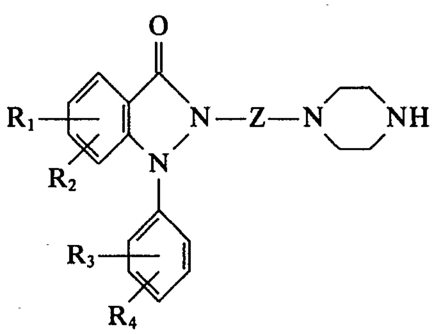

(III)

dans laquelle $R_1$, $R_2$, $R_3$, $R_4$ et Z ont la signification ci-dessus avec des composés de formule IV

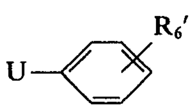

(IV)

dans laquelle $R_6'$ a la signification indiquée plus haut et U représente un halogène et que, le cas échéant, on transforme les composés libres de formule I en leurs sels d'addition avec des acides ou transforme les sels d'addition avec des acides en les composés libres de formule I.

12. Médicaments renfermant une quantité pharmacologiquement active d'un composé de 1-phénylindazol-3-one selon la revendication 1 et les adjuvants et/ou véhicules pharmaceutiques usuels.

**Revendications pour l'Etat contractant: AT**

1. Procédé pour la préparation de composés de 1-phénylindazol-3-one de formule générale I

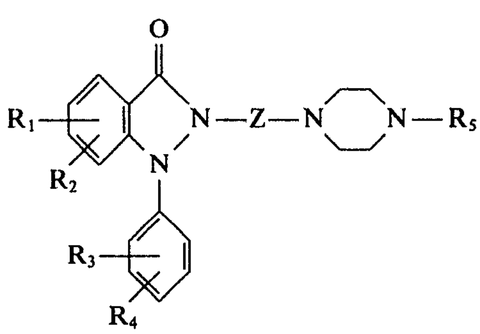

(I)

dans laquelle

$R_1$ représente de l'hydrogène, un alkyle avec 1 à 4 atomes de carbone, un alcoxy avec 1 à 4 atomes de carbone, un halogène ou un trifluorométhyle,
$R_2$ représente de l'hydrogène, un alkyle avec 1 à 4 atomes de carbone, un alcoxy avec 1 à 4 atomes de carbone ou un halogène,
$R_3$ représente de l'hydrogène, un alkyle avec 1 à 4 atomes de carbone, un alcoxy avec 1 à 4 atomes de carbone, un halogène ou un trifluorométhyle,
$R_4$ représente de l'hydrogène, un alkyle avec 1 à 4 atomes de carbone, un alcoxy avec 1 à 4 atomes de carbone ou un halogène,
Z représente un groupe alkylène avec 2 à 6 atomes de carbone,
$R_5$ représente un pyridyle qui est non substitué ou monosubstitué par un alkyle avec 1 à 4 atomes de carbone, un halogène ou un alcoxy avec 1 à 4 atomes de carbone, un thiényle ou un groupe phényle a éventuellement substitué

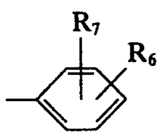

(a)

où

$R_6$ représente de l'hydrogène, un alkyle avec 1 à 4 atomes de carbone, un alcoxy avec 1 à 4 atomes de carbone, un hydroxy, un halogène, un trifluorométhyle ou un alcanoyloxy avec 1 à 4 atomes de carbone,
$R_7$ représente de l'hydrogène, un alkyle avec 1 à 4 atomes de carbone, un alcoxy avec 1 à 4 atomes de carbone ou un halogène ou
$R_6$ et $R_7$ sont fixés sur des atomes de carbone voisins et représentent ensemble un méthylènedioxy ou un éthylènedioxy,

ainsi que de leurs sels d'addition avec des acides, caractérisé en ce que

a) on fait réagir des composés de formule II a

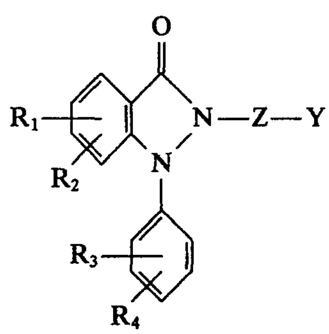

(II a)

dans laquelle $R_1$, $R_2$, $R_3$, $R_4$ et Z ont la signification ci-dessus et Y représente un reste éliminable par aminolyse, avec un composé de formule V

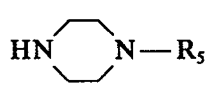

(V)

dans laquelle $R_5$ a la signification ci-dessus, ou que,

b) pour la préparation de composés de formule I a

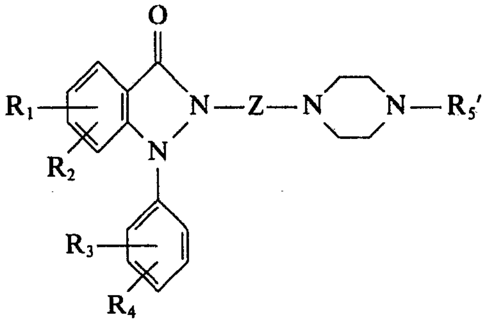

(Ia)

dans laquelle Z, $R_1$, $R_2$, $R_3$ et $R_4$ ont la signification ci-dessus et $R_5'$ est un reste phényle a' substitué

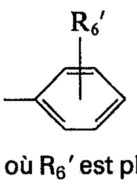

(a')

où $R_6'$ est placé en position ortho ou para et représente $CF_3$, on fait réagir des composés de formule III

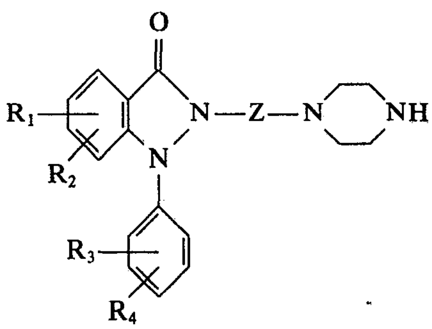

(III)

dans laquelle $R_1$, $R_2$, $R_3$, $R_4$ et Z ont la signification ci-dessus avec des composés de formule IV

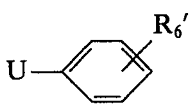

(IV)

dans laquelle $R_6'$ a la signification indiquée plus haut et U représente un halogène et que, le cas échéant, on transforme les composés libres de formule I en leurs sels d'addition avec des acides ou transforme les sels d'addition avec des acides en les composés libres de formule I.

2. Procédé selon la revendication 1, caractérisé en ce que l'on prépare, en tant que composés de formule I, des composés de 1-phénylindazol-3-one où Z, $R_1$, $R_2$, $R_3$ et $R_4$ ont la signification ci-dessus et $R_5$ est un groupe phényle a éventuellement substitué

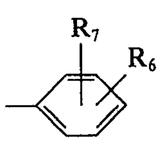

(a)

dans lequel $R_6$ et $R_7$ ont la signification ci-dessus

3. Procédé selon la revendication 1, caractérisé en ce que l'on prépare, en tant que composés de formule I, des composés de 1-phénylindazol-3-one selon la revendication 1 ou Z, $R_1$, $R_2$, $R_3$ et $R_4$ ont la signification ci-dessus et $R_5$ représente un pyridyle qui est non substitué ou monosubstitué par un alkyle avec 1 à 4 atomes de carbone, un halogène ou un alcoxy avec 1 à 4 atomes de carbone.

4. Procédé selon la revendication 2, caractérisé en ce que l'on prépare, en tant que composés de formule I, la 1-phényl-2-(4-[4-(4-fluorophényl)-pipérazin-1-yl]-butyl)-1,2-dihydro-3 H-indazol-3-one et ses sels d'addition avec des acides.

5. Procédé selon la revendication 2, caractérisé en ce que l'on prépare, en tant que composés de formule I, la 1-phényl-2-(4-[4-(2-méthoxyphényl)-pipérazin-1-yl]-butyl)-1,2-dihydro-3 H-indazol-3-one et ses sels d'addition avec des acides.

6. Procédé selon la revendication 3, caractérisé en ce que l'on prépare, en tant que composés de formule I, la 1-phényl-2-(4-[4-(2-pyridyl)-pipérazin-1-yl]-butyl)-1,2-dihydro-3 H-indazol-3-one et ses sels d'addition avec des acides.

7. Procédé selon la revendication 2, caractérisé en ce que l'on prépare, en tant que composés de formule I, la 1-(4-méthoxyphényl)-2-(4-[4-(4-fluorophényl)-pipérazin-1-yl]-butyl-1,2-dihydro-3 H-indazol-3-one et ses sels d'addition avec des acides.

8. Procédé pour la préparation de composés de formule générale II

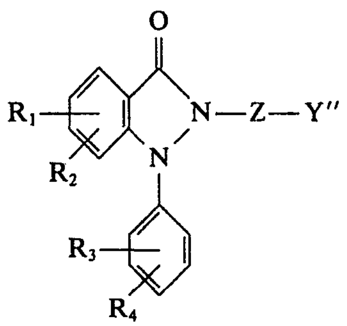

(II)

dans laquelle $R_1$, $R_2$, $R_3$, $R_4$ et Z ont la signification indiquée dans la revendication 1 et Y'' représente un reste éliminable par aminolyse ou un hydroxy, à l'exception des composés où $R_1$ à $R_4$ représentent chacun de l'hydrogène, Y'' représente du chlore et Z un éthylène ou un propylène et de leurs sels d'addition avec des acides, caractérisé en ce que l'on fait réagir des sels de métaux alcalins des composés de 1-phényl-1,2-dihydro-3 H-indazol-3-one de formule générale VI

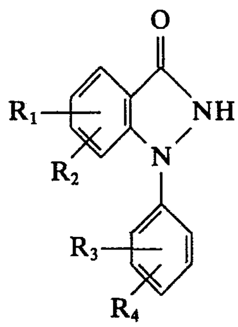

(VI)

dans laquelle $R_1$, $R_2$, $R_3$ et $R_4$ ont la signification ci-dessus avec des composés de formule générale VII

$$Y—Z—Y' \qquad (VII)$$

dans laquelle Y représente un reste éliminable par aminolyse et Y' représente un reste éliminable par aminolyse ou un groupe hydroxyle protégé et qu'on libère consécutivement un groupe hydroxyle éventuellement protégé par élimination du groupe protecteur.

9. Procédé selon la revendication 8, caractérisé en ce que l'on prépare, en tant que composés de formule II, des composés où $R_1$, $R_2$, $R_3$, $R_4$ et Z ont la signification indiquée plus haut et Y'' représente un halogène, un hydroxy, un alcanesulfonyloxy avec 1 à 4 atomes de carbone, un benzènesulfonyloxy ou un benzènesulfonyloxy substitué dans le noyau benzénique par un alkyle avec 1 à 4 atomes de carbone ou un halogène.

10. Procédé pour la préparation de composés de formule générale III

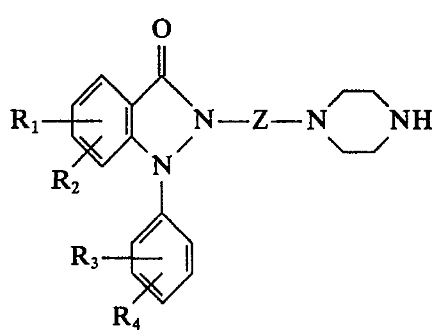

(III)

dans laquelle $R_1$, $R_2$, $R_3$, $R_4$ et Z ont la signification indiquée dans la revendication 1 et de leurs sels d'addition avec des acides, caractérisé en ce que l'on fait réagir des composés de formule générale II a

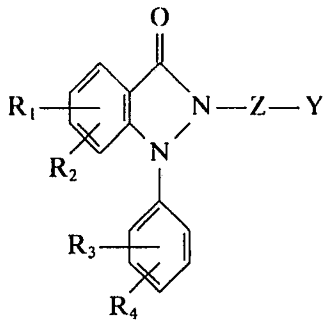

(II a)

dans laquelle $R_1$, $R_2$, $R_3$, $R_4$ et Z ont la signification ci-dessus et Y représente un reste éliminable par aminolyse, avec un excés de pipérazine ou que, à partir des composés de formule générale VIII

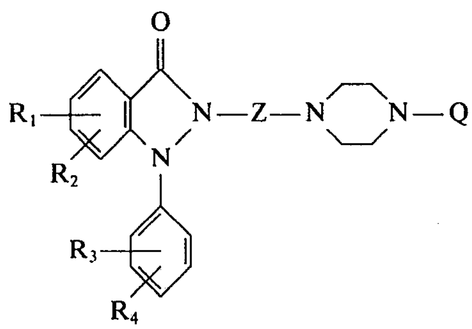

(VIII)

dans laquelle $R_1$, $R_2$, $R_3$, $R_4$ et Z ont la signification indiquée plus haut et Q représente un groupe amino protecteur, on sépare ce groupe amino protecteur.